# EUROPEAN PATENT APPLICATION

(11) **EP 4 810 613 A1**
(43) Date of publication of application: **23.09.2026**
(21) Application number: 24890645.5
(22) Date of filing: 12.11.2024
(51) Int. Cl.: C12N 15/62, A61K 39/25, A61P 31/22, A61P 25/00

(54) **MRNA VACCINE AGAINST HERPES ZOSTER AND PREPARATION METHOD THEREFOR**

(30) Priority: 13.11.2023 CN 202311503470
(71) Applicant: CSPC Megalith Biopharmaceutical Co., Ltd., Shijiazhuang, Hebei 050025 (CN)
(72) Inventor: FAN, Chao, Shijiazhuang, Hebei 050025 (CN); SU, Xiaoye, Shijiazhuang, Hebei 050025 (CN); WANG, Lingyu, Shijiazhuang, Hebei 050025 (CN); WEI, Lifan, Shijiazhuang, Hebei 050025 (CN); YANG, Sicong, Shijiazhuang, Hebei 050025 (CN); WU, Leibin, Shijiazhuang, Hebei 050025 (CN); LI, Chunlei, Shijiazhuang, Hebei 050025 (CN); YANG, Hanyu, Shijiazhuang, Hebei 050025 (CN); DAN, Mo, Shijiazhuang, Hebei 050025 (CN); ZHONG, Qiang, Shijiazhuang, Hebei 050025 (CN)
(74) Representative: Gille Hrabal Partnerschaftsgesellschaft mbB Patentanwälte
(86) International application number: PCT/CN2024/131485
(87) International publication number: WO 2025/103290

(57) **Abstract**

The present invention relates to the field of nucleic acid vaccines, and in particular to an mRNA vaccine against the herpes zoster virus and a preparation method therefor. The main components of the vaccine of the present invention comprise mRNA encoding the varicella zoster virus glycoprotein gE or a variant thereof and a lipid nanoparticle. The glycoprotein gE variant mRNA comprises a coding region encoding a VZV glycoprotein E (gE protein) extracellular domain and transmembrane domain, or a coding region composed of the extracellular domain and the transmembrane domain, and does not encode an intracellular domain of the gE protein; or comprises mRNA encoding VZV gE protein having a mutation site.

## Description

### CROSS-REFERENCE TO RELATED APPLICATIONS

The present disclosure claims priority to the prior application with the patent application No. 202311503470.5 entitled "MRNA VACCINE AGAINST HERPES ZOSTER AND PREPARATION METHOD THEREFOR" and filed with the China National Intellectual Property Administration on Nov. 13, 2023, which is incorporated herein by reference in its entirety.

### TECHNICAL FIELD

The present application relates to the technical field of biomedicine, and in particular to an mRNA vaccine against herpes zoster and a preparation method therefor.

### BACKGROUND

Herpes zoster (HZ) is an acute infectious skin disease caused by the reactivation of varicella-zoster virus (VZV) that is latent in the body. Primary VZV infection in children causes chickenpox. After infection, the virus enters the sensory nerve endings of the skin, moves centrally along nerve fibers of the dorsal root of the spinal cord or the trigeminal ganglion, and exists in a persistent latent form for a long time in the spinal nerve or cranial sensory ganglia. When the human body is immunocompromised or subjected to non-specific stimulation, the virus reactivates, grows, and reproduces, resulting in inflammation or necrosis of the affected ganglia and leading to neuralgia. Meanwhile, the reactivated virus spreads from one or more adjacent ganglia along the corresponding sensory nerve fibers to the skin, causing clustered blisters on the basis of unilateral distribution of erythema. The incidence of herpes zoster in the Asia-Pacific region is about 1%, which increases with age. The incidence of herpes zoster reaches 5% or more in patients over 40-50 years old. About 9%-34% of patients with herpes zoster can develop postherpetic neuralgia, which has a pain grade reaching grade 7 or higher, belongs to severe pain, and seriously affects the quality of life of the patients. There is no specific medicine for herpes zoster and postherpetic neuralgia, and thus, vaccination against herpes zoster is an effective means for preventing herpes zoster.

Commercially available herpes zoster vaccine products in the world include Zostavax (Merck Sharp & Dohme), Shingrix (GSK), SkyZoster (SK), and Ganwei (BCHT). Zostavax and SkyZoster are live attenuated vaccines that achieve structural changes and attenuated toxicity while retaining immunogenicity through treatment, thereby establishing immune responses; Shingrix is a recombinant subunit vaccine that generates and enhances immune responses *in vivo* through the varicella-zoster glycoprotein E antigen and AS01B adjuvant. Clinical data have shown that the efficacy of Shingrix is 97% for patients aged 50 to 69 years, and the efficacy is still as high as 91% for patients aged 70 years or more. The efficacy of Zostavax is 70% for patients aged 50 to 69 years, and the overall efficacy is 51%. The effectiveness of Shingrix is superior to that of Zostavax. A safer and more effective VZV vaccine is still urgently needed in the market.

### SUMMARY

In order to meet the diversified needs of the market for vaccines against the varicella-zoster virus by multiple technical routes, the present application provides an immune composition (e.g., an mRNA vaccine) comprising an RNA encoding a highly immunogenic antigen capable of eliciting an effective neutralizing antibody response and cellular immunity against the varicella-zoster virus. In the present disclosure, a VZV mRNA vaccine is prepared based on gE glycoprotein on the surface of the varicella-zoster virus (VZV). An mRNA encoding an antigenic polypeptide, an immunogenic fragment, or a variant thereof of the varicella-zoster virus VZV is used, and the lipid nanoparticle (LNP) technology is selected to encapsulate the mRNA and deliver it to immune cells *in vivo*. LNPs release the mRNA within the cells, and the mRNA is translated into an immunogenic fragment or a variant thereof of the varicella-zoster virus (VZV), which is referred to as "VZV antigen peptide" or "antigen peptide" in the present application. The VZV antigen peptide expressed by immune cells further stimulates the humoral immunity of the organism and generates neutralizing antibodies against the VZV virus, thereby achieving the protective effect. The mRNA vaccine can stimulate the humoral immunity and stimulate the cellular immunity against the virus at the same time, and the activated T cells can kill virus-infected cells and generate memory cells to continuously maintain the protective efficacy.

Specifically, the present application provides the following optional embodiments.

In a first aspect, the present application provides a nucleic acid molecule encoding a varicella-zoster virus (VZV) glycoprotein or a variant thereof, wherein the VZV glycoprotein is selected from: VZV gE, gI, gB, gH, gK, gL, gC, gN, and gM.

In some embodiments, the nucleic acid molecule comprises a VZV RNA polynucleotide having an open reading frame encoding a VZV gE protein or a variant thereof (which belongs to the VZV antigen peptide).

In some embodiments, the amino acid sequence of the VZV gE protein variant comprises one or more mutations selected from the following relative to SEQ ID NO. 8, or the mutation relative to SEQ ID NO. 8 is one or more mutations selected from the following: S593L, S595L, T596L, H362E, T598L, S593I, S595I, T596I, T598I, and Del 560-623, wherein the positions of the amino acids are numbered according to the amino acid sequence of SEQ ID NO. 8 as a reference sequence; for example, S593L indicates a mutation from S to L at position 593 relative to the reference sequence SEQ ID NO: 8.

In some embodiments, the amino acid sequence of the VZV gE protein variant comprises or has only any one of the following mutations relative to SEQ ID NO. 8:
1) S593L, S595L, T596L, and T598L;
2) H362E, S593L, S595L, T596L, and T598L;
3) H362E, S593I, S595I, T596I, and T598I;
4) S593I, S595I, T596I, and T598I; and
5) Del 560-623;
and wherein the positions of the amino acids are numbered according to the amino acid sequence of SEQ ID NO. 8 as a reference sequence.

In some embodiments, the VZV gE protein variant does not comprise a signal peptide of the VZV gE protein, and the amino acid sequence of a portion thereof other than the signal peptide comprises or has only any one of the following mutations relative to SEQ ID NO. 8:
1) S593L, S595L, T596L, and T598L;
2) H362E, S593L, S595L, T596L, and T598L;
3) H362E, S593I, S595I, T596I, and T598I;
4) S593I, S595I, T596I, and T598I; and
5) Del 560-623;
and wherein the positions of the amino acids are numbered according to the amino acid sequence of SEQ ID NO. 8 as a reference sequence.

In some embodiments, the VZV gE protein variant comprises a signal peptide of a VZV gE protein, and the amino acid sequence of a portion thereof other than the signal peptide comprises or has only any one of the following mutations relative to SEQ ID NO. 8:
1) S593L, S595L, T596L, and T598L;
2) H362E, S593L, S595L, T596L, and T598L;
3) H362E, S593I, S595I, T596I, and T598I;
4) S593I, S595I, T596I, and T598I; and
5) Del 560-623;
and wherein the positions of the amino acids are numbered according to the amino acid sequence of SEQ ID NO. 8 as a reference sequence.

In some embodiments, the VZV gE protein variant comprises an extracellular region and a transmembrane region of VZV glycoprotein E (which may be referred to as gE protein or gE for short) or consists of an extracellular region and a transmembrane region of VZV glycoprotein E, but does not encode an intracellular region of the gE protein.

In some embodiments, the extracellular region comprises amino acids at positions 31-538 of the varicella-zoster virus gE protein, the transmembrane region comprises amino acids at positions 539-559 of the varicella-zoster virus gE protein, and the positions of the amino acids are numbered according to the amino acid sequence of SEQ ID NO. 8 as a reference sequence.

In some embodiments, the VZV gE protein variant comprises an extracellular region and a transmembrane region of the VZV gE protein, but does not comprise an intracellular region, wherein the extracellular region comprises amino acids at positions 31-538 of the VZV gE protein; the transmembrane region comprises amino acids at positions 539-559 of the VZV gE protein; and the amino acid sequence of the gE protein is set forth in SEQ ID NO. 8.

In some specific embodiments, the antigen peptide further comprises a signal peptide sequence, and the signal peptide sequence is located at the N-terminus of the amino acid sequence of the extracellular region.

In some embodiments, the signal peptide is a signal peptide of the VZV gE protein.

In some embodiments, the signal peptide comprises amino acids at positions 1-30 or 2-30 of SEQ ID NO. 8.

In some embodiments, the signal peptide is not a natural signal peptide of the VZV gE protein.

In some embodiments, the signal peptide is a signal peptide derived from other viruses that can infect mammalian cells or a signal peptide derived from other mammalian proteins.

In some embodiments, the signal peptide is a signal peptide derived from envelope proteins of other envelope viruses.

In some embodiments, the VZV gE protein variant comprises an extracellular region and a transmembrane region of the VZV gE protein, but does not comprise an intracellular region, wherein the extracellular region comprises amino acids at positions 31-538 of the VZV gE protein; the transmembrane region comprises amino acids at positions 539-559 of the VZV gE protein; the intracellular region comprises amino acids at positions 560-623 of the VZV gE protein; the amino acid sequence of the gE protein is set forth in SEQ ID NO. 8; and the VZV gE protein variant further comprises a signal peptide, which is not a natural signal peptide of the VZV gE protein.

In some embodiments, the VZV gE protein variant comprises an extracellular region and a transmembrane region of the VZV gE protein, but does not comprise an intracellular region, wherein the extracellular region comprises amino acids at positions 31-538 of the VZV gE protein; the transmembrane region comprises amino acids at positions 539-559 of the VZV gE protein; the intracellular region comprises amino acids at positions 560-623 of the VZV gE protein; the amino acid sequence of the gE protein is set forth in SEQ ID NO. 8; and the VZV gE protein variant further comprises a signal peptide, which is not a natural signal peptide of the VZV gE protein, but is a signal peptide derived from other viruses that can infect mammalian cells or a signal peptide derived from other mammalian proteins.

In some embodiments, the VZV gE protein variant comprises an extracellular region of the VZV gE protein, a transmembrane region of the VZV gE protein, and a signal peptide of the VZV gE protein, but does not comprise an intracellular region of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; the extracellular region, the transmembrane region, and the signal peptide are each independently linked by 0, 1, or more amino acid residues; and the positions of the amino acids are numbered according to the amino acid sequence of SEQ ID NO. 8 as a reference sequence.

In some embodiments, the VZV gE protein variant comprises an extracellular region of the VZV gE protein, a transmembrane region of the VZV gE protein, and a signal peptide of the VZV gE protein, but does not comprise an intracellular region of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; from the N-terminus to the C-terminus, the extracellular region, the transmembrane region, and the signal peptide are each independently linked by 0, 1, or more amino acid residues; and the positions of the amino acids are numbered according to the amino acid sequence of SEQ ID NO. 8 as a reference sequence.

In some embodiments, the VZV gE protein variant comprises an extracellular region of the VZV gE protein, a transmembrane region of the VZV gE protein, and a signal peptide of the VZV gE protein, but does not comprise an intracellular region of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; the extracellular region, the transmembrane region, and the signal peptide are each independently linked by 0, 1, or more amino acid residues; and the amino acid sequence of the VZV gE protein is set forth in SEQ ID NO. 8.

In some embodiments, the VZV gE protein variant comprises an extracellular region of the VZV gE protein, a transmembrane region of the VZV gE protein, and a signal peptide of the VZV gE protein, but does not comprise an intracellular region of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; from the N-terminus to the C-terminus, the extracellular region, the transmembrane region, and the signal peptide are each independently linked by 0, 1, or more amino acid residues; and the amino acid sequence of the VZV gE protein is set forth in SEQ ID NO. 8.

In some embodiments, the VZV gE protein variant comprises an extracellular region, a transmembrane region, and an intracellular region of the VZV gE protein, wherein the extracellular region comprises amino acids at positions 31-538 of the VZV gE protein; the transmembrane region comprises amino acids at positions 539-559 of the VZV gE protein; the intracellular region comprises amino acids at positions 560-623 of the VZV gE protein; the amino acid sequence of the gE protein is set forth in SEQ ID NO. 8; and the VZV gE protein variant further comprises a signal peptide, which is not a natural signal peptide of the VZV gE protein.

In some embodiments, the VZV gE protein variant comprises an extracellular region, a transmembrane region, and an intracellular region of the VZV gE protein, wherein the extracellular region comprises amino acids at positions 31-538 of the VZV gE protein; the transmembrane region comprises amino acids at positions 539-559 of the VZV gE protein; the intracellular region comprises amino acids at positions 560-623 of the VZV gE protein; the amino acid sequence of the gE protein is set forth in any one of SEQ ID NOs. 14-17; and the VZV gE protein variant further comprises a signal peptide, which is not a natural signal peptide of the VZV gE protein, but is a signal peptide derived from other viruses that can infect mammalian cells or a signal peptide derived from other mammalian proteins.

In some embodiments, the VZV gE protein variant comprises an extracellular region of the VZV gE protein, a transmembrane region of the VZV gE protein, an intracellular region of the VZV gE protein, and a signal peptide of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; the extracellular region, the transmembrane region, the intracellular region, and the signal peptide are each independently linked by 0, 1, or more amino acid residues; and the positions of the amino acids are numbered according to the amino acid sequence of SEQ ID NO. 8 as a reference sequence.

In some embodiments, the VZV gE protein variant comprises an extracellular region of the VZV gE protein, a transmembrane region of the VZV gE protein, an intracellular region of the VZV gE protein, and a signal peptide of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; from the N-terminus to the C-terminus, the extracellular region, the transmembrane region, the intracellular region, and the signal peptide are each independently linked by 0, 1, or more amino acid residues; and the positions of the amino acids are numbered according to the amino acid sequence of SEQ ID NO. 8 as a reference sequence.

In some embodiments, the VZV gE protein variant comprises an extracellular region of the VZV gE protein, a transmembrane region of the VZV gE protein, an intracellular region of the VZV gE protein, and a signal peptide of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; the extracellular region, the transmembrane region, the intracellular region, and the signal peptide are each independently linked by 0, 1, or more amino acid residues; and the amino acid sequence of the VZV gE protein is set forth in any one of SEQ ID NOs. 14-17.

In some embodiments, the VZV gE protein variant comprises an extracellular region of the VZV gE protein, a transmembrane region of the VZV gE protein, an intracellular region of the VZV gE protein, and a signal peptide of the VZV gE protein, wherein the extracellular region comprises amino acids at positions 31-538 of the VZV gE protein; the transmembrane region comprises amino acids at positions 539-559 of the VZV gE protein; the signal peptide comprises amino acids at positions 1-30 or 2-30 of the VZV gE protein; the intracellular region comprises amino acids at positions 560-623 of the VZV gE protein; from the N-terminus to the C-terminus, the extracellular region, the transmembrane region, the intracellular region, and the signal peptide are each independently linked by 0, 1, or more amino acid residues; and the amino acid sequence of the VZV gE protein is set forth in any one of SEQ ID NOs. 14-17.

In some embodiments, the gE protein or the variant thereof comprises amino acid sequence having at least 80%, at least 85%, at least 90%, at least 95%, at least 98%, at least 99%, or 100% identity to the amino acid sequence set forth in any one of SEQ ID NOs. 13-17, or the amino acid sequence of the antigen has at least 80%, at least 85%, at least 90%, at least 95%, at least 98%, at least 99%, or 100% identity to the amino acid sequence set forth in any one of SEQ ID NOs. 13-17.

In some embodiments, the amino acid sequence of the gE protein or the variant thereof is set forth in any one of SEQ ID NOs. 13-17.

In some embodiments, the amino acid sequence of the gE protein or the variant thereof is set forth in SEQ ID NO. 13.

In some embodiments, a sequence (coding sequence) encoding the aforementioned antigen peptide in the nucleic acid molecule is codon-optimized to increase the GC content.

In some embodiments, a sequence (coding sequence) encoding the aforementioned antigen peptide in the nucleic acid molecule is codon-optimized so that codons therein are high-frequency codons in mammalian cells.

In some embodiments, a sequence (coding sequence) encoding the aforementioned antigen peptide in the nucleic acid molecule is codon-optimized so that codons therein are high-frequency codons in human cells.

In some embodiments, a sequence (coding sequence) encoding the aforementioned antigen peptide in the nucleic acid molecule is codon-optimized so that codons therein are high-frequency codons in human nerve cells.

In some embodiments, a sequence (coding sequence) encoding the aforementioned antigen peptide in the nucleic acid molecule is codon-optimized so that codons therein are high-frequency codons in human immune cells.

In some embodiments, the aforementioned nucleic acid molecule is a DNA.

In some embodiments, the aforementioned nucleic acid molecule is an RNA.

In some embodiments, the nucleic acid molecule is an mRNA, a self-replicating RNA, a circular RNA, or a replicon RNA, preferably an mRNA.

In some embodiments, the mRNA molecule comprises at least the following structures:
(a) a 5'-cap structure;
(b) a 5' UTR having a length of 10-200 nucleotides, preferably 15-150 nucleotides;
(c) a 3' polyadenylic acid sequence;
(d) a 3' UTR.

In some embodiments, the aforementioned mRNA molecule comprises a 5'-cap structure, wherein the 5'-cap structure is an m7G structure, a cap0 structure, a cap1 structure, a cap2 structure, a modified cap0 structure, or a modified cap1 structure; the 5'-cap structure is preferably an m7G structure, a cap0 structure, a cap1 structure, a cap2 structure, a modified cap0 structure, or a modified cap1 structure, which is selected from any one of the following: m7Gppp(2'OMeA)pG, m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG, m7GpppUpU, m7Gpppm6ApG, m7G_{3'Ome}pppApA, m7G_{3'Ome}pppApC, m7G_{3'Ome}pppApU, m7G_{3'Ome}pppApG, m7G_{3'Ome}pppCpA, m7G_{3'Ome}pppCpC, m7G_{3'Ome}pppCpG, m7G_{3'Ome}pppCpU, m7G_{3'Ome}pppUpA, m7G_{3'Ome}pppUpC, m7G_{3'Ome}pppUpG, m7G_{3'Ome}pppUpU, m7G_{3'Ome}PppA_{2'Ome}pG, m7G_{3'Ome}pppA_{2'Ome}pC, m7G_{3'Ome}pppA_{2'Ome}pU, m7G_{3'Ome}pppA_{2'Ome}pA, m7G_{3'Ome}pppC_{2'Ome}pA, m7G_{3'Ome}pppC_{2'Ome}pU, m7G_{3'Ome}pppC_{2'Ome}pG, m7G_{3'Ome}pppC_{2'Ome}pC, m7G_{3'Ome}pppG_{2'Ome}pA, m7G_{3'Ome}pppG_{2'Ome}pU, m7G_{3'Ome}pppG_{2'Ome}pG, m7G_{3'Ome}pppG_{2'Ome}pC, m7G_{3'Ome}pppU_{2'Ome}pA, m7G_{3'Ome}pppU_{2'Ome}pU, m7G_{3'Ome}pppU_{2'Ome}pG, and m7G_{3'Ome}pppU_{2'Ome}pC, most preferably m7G(5')ppp(5')(2'OMeA)pG.

In some embodiments, the mRNA molecule comprises a 5' UTR or a coding sequence of the 5' UTR, wherein the 5' UTR is selected from *Xenopus laevis* or human α-globin or β-globin, human cytochrome b-245a polypeptide, hydroxysteroid (17b) dehydrogenase, tobacco etch virus, α-1-globin, Kozak sequence, HSD17B4, RPL32, ASAH1, ATP5A1, MP68, NDUFA4, NOSIP, RPL31, SLC7A3, TUBB4B, and UBQLN2, or a homolog, fragment, or variant from any one of these genes;
preferably, the 5' UTR comprises or is a tobacco etch virus (TEV) 5' UTR; further preferably, the nucleotide sequence of the TEV 5' UTR comprises the nucleotide sequence set forth in SEQ ID NO. 18 or has at least 100%, 99%, 98%, 97%, 96%, 95%, 90%, 85%, or 80% sequence identity to the nucleotide sequence set forth in SEQ ID NO: 18;
   or,
the nucleotide sequence of the 5' UTR comprises a Kozak sequence having the nucleotide sequence set forth in SEQ ID NO. 9 or has at least 100%, 99%, 98%, 97%, 96%, 95%, 90%, 85%, or 80% sequence identity to the nucleotide sequence set forth in SEQ ID NO: 9;
   or,
the nucleotide sequence of the 5' UTR is pVAX.1+TEV set forth in SEQ ID NO. 10 or has at least 100%, 99%, 98%, 97%, 96%, 95%, 90%, 85%, or 80% sequence identity to the nucleotide sequence set forth in SEQ ID NO: 10.

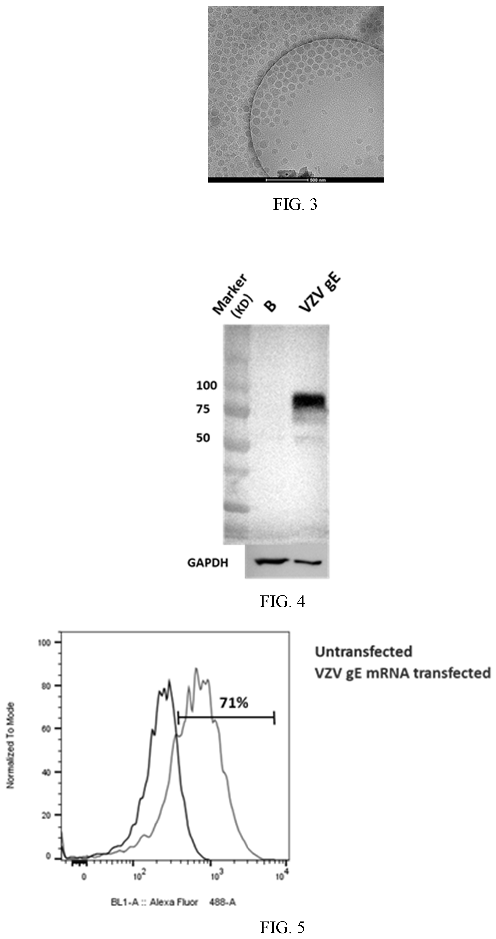

In some embodiments, the mRNA molecule comprises a 3' UTR or a coding sequence of the 3' UTR, wherein the 3' UTR may also be selected from 3' UTRs of PSMB3, ALB7, α-globin, CASP1, COX6B1, GNAS, NDUFA1, DH143, gp130, hHBB, hHBA1, CYBA (cytochrome b-245 alpha chain), rabbit β-globin, hepatitis B virus (HBV), VEEV (Venezuelan equine encephalitis virus) virus, rps9 (ribosomal protein S9), FIG4 (FIG4 phosphoinositide 5-phosphatase), human albumin hHBB (human hemoglobin subunit beta), and HBA1 (human hemoglobin subunit alpha 1), or a homolog, fragment, or variant from any one of these genes;
preferably, the 3' UTR comprises or is a hemoglobin-1 (hHBA1) 3' UTR; further preferably, the nucleotide sequence of the hHBA1 3' UTR comprises the nucleotide sequence set forth in SEQ ID NO. 12 or has at least 100%, 99%, 98%, 97%, 96%, 95%, 90%, 85%, or 80% sequence identity to the nucleotide sequence set forth in SEQ ID NO: 12.

In some embodiments, the mRNA molecule comprises 3' poly(A) or a coding sequence of the 3' poly(A), wherein the 3' poly(A) is 90-120 nt in length and comprises a base other than A; preferably, the nucleotide sequence of the 3' poly(A) comprises the nucleotide sequence set forth in SEQ ID NO. 11 or has at least 100%, 99%, 98%, 97%, 96%, 95%, 90%, 85%, or 80% sequence identity to the nucleotide sequence set forth in SEQ ID NO: 11.

In some embodiments, part or all of uridines (U) in the mRNA molecule are base-modified uridines or uridine analogs; preferably, the chemically modified uridine is any one or more selected from the group consisting of 5-methoxymethyl uridine, 5-methylthio uridine, 1-methoxymethyl pseudouridine, 5-methyl cytidine, 5-methoxy cytidine, 1-methyl-pseudouridine (N1-methyl-pseudo-UTP), and pseudouridine; more preferably, each U in the sequence is 1-methyl-pseudouridine.

In some embodiments, the mRNA molecule comprises or consists of a nucleotide sequence having at least 80%, at least 85%, at least 90%, at least 95%, at least 98%, at least 99%, or 100% identity to the nucleotide sequence set forth in any one of SEQ ID NOs. 1-7.

In some embodiments, the mRNA molecule comprises or consists of a nucleotide sequence having at least 80%, at least 85%, at least 90%, at least 95%, at least 98%, at least 99%, or 100% identity to the nucleotide sequence set forth in any one of SEQ ID NOs. 2-7.

In some embodiments, the mRNA molecule comprises or consists of a nucleotide sequence having at least 80%, at least 85%, at least 90%, at least 95%, at least 98%, at least 99% or 100% identity to the nucleotide sequence set forth in SEQ ID NO. 3.

In some embodiments, the mRNA molecule is formed by performing capping on a nucleotide sequence having at least 80%, at least 85%, at least 90%, at least 95%, at least 98%, at least 99%, or 100% identity to the nucleotide sequence set forth in any one of SEQ ID NOs. 1-7.

In some embodiments, the mRNA molecule is formed by performing capping on a nucleotide sequence having at least 80%, at least 85%, at least 90%, at least 95%, at least 98%, at least 99%, or 100% identity to the nucleotide sequence set forth in any one of SEQ ID NOs. 2-7.

In some embodiments, the mRNA molecule is formed by performing capping on a nucleotide sequence having at least 80%, at least 85%, at least 90%, at least 95%, at least 98%, at least 99%, or 100% identity to the nucleotide sequence set forth in SEQ ID NO. 3.

In a second aspect, the present application provides a composition, preferably an immunogenic composition, comprising at least one nucleic acid molecule according to the first aspect. Appropriately, the composition may comprise at least one nucleic acid molecule, e.g., at least one coding RNA, and the nucleic acid is complexed with one or more lipids, encapsulated in one or more lipids, or associated with one or more lipids, thereby forming a lipid nanoparticle.

In some embodiments, the composition relates to a nucleic acid vaccine against the varicella-zoster virus VZV, wherein the vaccine carrier is a lipid nanoparticle (LNP) comprising an ionizable cationic lipid, a structural lipid, a helper lipid, and a surfactant.

In some embodiments, the total molar content of the ionizable cationic lipid, the structural lipid, the helper lipid, and the surfactant is 100% by molar percentage (mol%).

In some embodiments, the lipid nanoparticle comprises 20-60 mol% ionizable cationic lipid, 25-55 mol% structural lipid, 5-25 mol% helper lipid, and 0.5-15 mol% surfactant.

In some embodiments, the cationic lipid is selected from SM-102, ALC-0315, ALC-0519, Dlin-MC3-DMA, DODMA, C12-200, and DlinDMA, preferably SM-102; the structure of SM-102 is as follows:

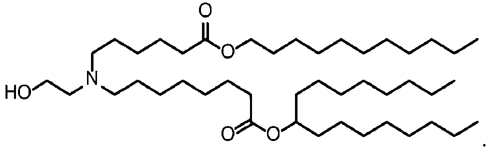

In some embodiments, the structural lipid is selected from cholesterol and a cholesterol derivative, preferably cholesterol.

In some embodiments, the helper lipid is selected from DSPC, DOPE, DOPC, DOPG, and DOPS, preferably DSPC.

In some embodiments, the surfactant is selected from PEG2000-DMG, PEG-DSPE, DTDA-PEG2000, and TPGS, preferably PEG2000-DMG.

In some embodiments, the lipid nanoparticle comprises 20-50 mol% ionizable cationic lipid. For example, the lipid nanoparticle may comprise 41, 42, 43, 44, 45, 46, 47, 48, 49, or 50 mol% ionizable cationic lipid.

In some other embodiments, the lipid nanoparticle comprises 50-60 mol% ionizable cationic lipid. For example, the lipid nanoparticle may comprise 51, 52, 53, 54, 55, 56, 57, 58, 59, or 60 mol% ionizable cationic lipid.

In some embodiments, the lipid nanoparticle comprises 5-25 mol% DSPC, preferably 2-15 mol% DSPC; for example, the lipid nanoparticle may comprise 3, 4, 5, 6, 7, 8, 9, 10, 11, 12, 13, 14, or 15 mol% DSPC.

In some embodiments, the lipid nanoparticle comprises 25-55 mol% cholesterol, preferably 30-40 mol% cholesterol. For example, the lipid nanoparticle may comprise 30, 31, 32, 33, 34, 35, 36, 37, 38, 39, or 40 mol% cholesterol.

In some embodiments, the lipid nanoparticle comprises 0.5-15 mol% DMG-PEG, preferably 1-2 mol% DMG-PEG. For example, the lipid nanoparticle may comprise 1, 1.5, or 2 mol% DMG-PEG. In some embodiments, the lipid nanoparticle comprises 50 mol% ionizable cationic lipid, 10 mol% DSPC, 38.5 mol% cholesterol, and 1.5 mol% DMG-PEG.

In some embodiments, the lipid nanoparticle comprises 50 mol% SM-102, 10 mol% DSPC, 38.5 mol% cholesterol, and 1.5 mol% DMG-PEG.

In some embodiments, the lipid nanoparticle of the present application comprises an N:P ratio of about 2:1 to about 30:1.

In some embodiments, the lipid nanoparticle of the present application comprises an N:P ratio of about 6:1.

In some embodiments, the lipid nanoparticle of the present application comprises an N:P ratio of about 3:1.

In some embodiments, the lipid nanoparticle of the present application comprises an ionizable cationic lipid component and an RNA in a wt/wt ratio of about 10:1 to about 100:1.

In some embodiments, the lipid nanoparticle of the present application comprises an ionizable cationic lipid component and an RNA in a wt/wt ratio of about 20:1.

In some embodiments, the lipid nanoparticle of the present application comprises an ionizable cationic lipid component and an RNA in a wt/wt ratio of about 10:1. In some embodiments, the lipid nanoparticle composition of the present application has an average diameter of about 50 nm to about 150 nm.

In some embodiments, the lipid nanoparticle of the present application has an average diameter of about 70 nm to about 120 nm, preferably 100-120 nm, and most preferably 100 nm.

In some embodiments, the mRNA solution is diluted with water for injection.

In some embodiments, the lipid nanoparticle and the mRNA are in a mass ratio of 1:1 to 30:1. The lipid carrier is preferably an LNP composition, and the mass ratio is preferably 20:1.

In some embodiments, the varicella-zoster virus nucleic acid vaccine of the present application further comprises: a buffer component and a cryoprotectant.

In some embodiments, the buffer may be selected from: but are not limited to, a citrate buffer, an acetate buffer, a phosphate buffer, ammonium chloride, calcium carbonate, calcium chloride, calcium citrate, calcium gluconate, calcium glycerophosphate, calcium lactate, calcium lactobionate, propionic acid, calcium levulinate, valeric acid, dicalcium phosphate, phosphoric acid, tricalcium phosphate, potassium acetate, potassium chloride, potassium gluconate, a potassium mixture, dipotassium phosphate, dibasic potassium phosphate, a potassium phosphate mixture, sodium acetate, sodium bicarbonate, sodium chloride, sodium citrate, sodium lactate, disodium hydrogen phosphate, sodium dihydrogen phosphate, sodium phosphate, magnesium hydroxide, aluminum hydroxide, alginic acid, polyethylene glycol, sodium benzoate, leucine, magnesium lauryl sulfate, sodium dodecyl sulfate, and combinations thereof.

In some embodiments, the cryoprotectant may be selected from substances such as sugars/polyols, polymers, surfactants, amino acids, and salts, wherein the sugars may be selected from: lactose, sucrose, trehalose, galactose, and the like.

In some embodiments, the amount of the cryoprotectant is 1 to 50% w/w, such as from 2 to 50% w/w, or from 4 to 45% w/w, or from 6 to 12% w/w, or preferably from 6 to 10% w/w, or most preferably from 7 to 9% w/w.

In some embodiments, the pharmaceutical composition of the present application comprises the aforementioned lipid nanoparticle and an external phase buffer.

In some embodiments, the aqueous phase buffer includes: tromethamine, sodium acetate, and sucrose, with a pH of 7-8.

In some embodiments, the content of tromethamine is selected from 10-30 mmol/L, preferably 15-25 mmol/L, preferably 15 mmol/L, 15.5 mmol/L, 16 mmol/L, 16.5 mmol/L, 17 mmol/L, 17.5 mmol/L, 18 mmol/L, 18.5 mmol/L, 19 mmol/L, 19.5 mmol/L, 20 mmol/L, 20.5 mmol/L, 21 mmol/L, 21.5 mmol/L, 22 mmol/L, 22.5 mmol/L, 23 mmol/L, 23.5 mmol/L, 24 mmol/L, 24.5 mmol/L, and 25 mmol/L, and most preferably 20 mmol/L.

In some embodiments, the content of sodium acetate is selected from 0-20 mmol/L, preferably 5-11 mmol/L, preferably 5 mmol/L, 5.5 mmol/L, 6 mmol/L, 6.5 mmol/L, 7 mmol/L, 7.5 mmol/L, 8 mmol/L, 8.5 mmol/L, 9 mmol/L, 9.5 mmol/L, 10 mmol/L, 10.5 mmol/L, 10.6 mmol/L, 10.7 mmol/L, 10.8 mmol/L, 10.9 mmol/L, 11 mmol/L, 11.5 mmol/L, 12 mmol/L, 12.5 mmol/L, and 13 mmol/L, and most preferably 10.7 mmol/L.

In some embodiments, the content of sucrose is selected from 5%-15%, preferably 7.5%-10%, more preferably 7.6%, 7.7%, 7.8%, 7.9%, 8.0%, 8.1%, 8.2%, 8.3%, 8.4%, 8.5%, 9%, 9.5%, and 10%, and most preferably 8.7%.

In some embodiments, the pharmaceutical composition of the present application comprises an mRNA having the sequence set forth in any one of SEQ ID NOs. 1-7, a lipid nanoparticle, 20 mmol/L tromethamine, 10.7 mmol/L sodium acetate, and 8.7% sucrose, with a pH of 7.0-8.0, wherein the lipid nanoparticle comprises 50 mol% SM-102, 10 mol% DSPC, 38.5 mol% cholesterol, and 1.5 mol% PEG2000-DMG, at N:P of 6.

In a third aspect, the present application provides a varicella-zoster virus antigen peptide.

In some embodiments, the antigen peptide is encoded by the nucleic acid molecule according to the first aspect.

In some embodiments, the amino acid sequence of the antigen peptide is identical to the amino acid sequence encoded by the nucleic acid molecule according to the first aspect.

In some embodiments, the antigen peptide comprises or consists of an amino acid sequence having at least 75%, at least 80%, at least 90%, at least 95%, at least 96%, at least 97%, at least 98%, at least 99%, or 100% identity to any one of SEQ ID NOs. 13-17.

In a fourth aspect, the present application provides a varicella-zoster virus vaccine, wherein the vaccine comprises at least one nucleic acid molecule according to the first aspect, or at least one composition according to the second aspect, or at least one antigen peptide according to the third aspect.

In some embodiments, the vaccine is a varicella-zoster virus (VZV) RNA (e.g., mRNA) vaccine comprising a coding sequence of a VZV gE antigen peptide, wherein the antigen peptide comprises, from the N-terminus to the C-terminus, a signal peptide sequence of the VZV gE protein, an extracellular region sequence of the VZV gE protein, and a transmembrane region sequence of the VZV gE protein in sequence.

In some embodiments, the VZV RNA (e.g., mRNA) vaccine comprises at least one ribonucleic acid (RNA) polynucleotide having an open reading frame encoding the VZV gE antigen peptide.

In some embodiments, the route of administration of the vaccine includes intravenous injection, intramuscular injection, or subcutaneous injection, preferably intramuscular injection.

In some embodiments, the dosage form of the vaccine may be selected from a lyophilized powder injection, a liquid dosage form for injection, and an inhalation formulation.

In a fifth aspect, the present application provides a kit or kit-of-parts comprising at least one nucleic acid molecule according to the first aspect, and/or at least one composition according to the second aspect, and/or at least one antigen peptide according to the third aspect, and/or at least one vaccine according to the fourth aspect.

In a sixth aspect, the present application provides a composition comprising at least two isolated components, wherein the at least two isolated components are selected from two nucleic acid molecules according to the first aspect, and/or two compositions according to the second aspect, and/or two antigen peptides according to the third aspect, and/or at least two vaccines according to the fourth aspect.

In a seventh aspect, the present application provides a method for treating or preventing a varicella-zoster virus infection in a subject, comprising administering to the subject at least one nucleic acid molecule according to the first aspect, and/or at least one composition according to the second aspect, and/or at least one antigen peptide according to the third aspect, and/or at least one vaccine according to the fourth aspect, which can effectively induce a neutralizing antibody response against the varicella-zoster virus (VZV) in the subject.

In an eighth aspect, the present application provides use of the nucleic acid molecule according to the first aspect, the composition according to the second aspect, the antigen peptide or protein according to the third aspect, or the vaccine according to the fourth aspect for manufacturing a medicament for the treatment or prevention of a varicella-zoster virus infection.

In a ninth aspect, the present application relates to a preparation method for a varicella-zoster virus vaccine, comprising mixing a vaccine carrier with the mRNA according to the first aspect to obtain the varicella-zoster virus vaccine.

In some embodiments, the vaccine carrier is a cationic lipid nanoparticle, and the preparation method comprises the following specific steps:
(1) dissolving an ionizable cationic lipid, a structural lipid, a helper lipid, and a surfactant in an organic solution according to the formula proportion to obtain an organic phase;
(2) dissolving the mRNA described above in a citrate buffer or a sodium acetate solution to obtain an aqueous phase; and
(3) uniformly mixing the organic phase in step (1) and the aqueous phase in step (2) to produce a mixed solution, so as to obtain the varicella-zoster virus vaccine.

In some embodiments, the organic solution includes absolute ethanol.

In some embodiments, the total concentration of the ionizable cationic lipid, the structural lipid, the helper lipid, and the surfactant in the organic phase is 10-15 mg/mL.

In some embodiments, the concentration of the mRNA is 0.01-1 mg/mL, preferably 0.1-0.2 mg/mL. In some embodiments, the volume ratio of the organic phase to the aqueous phase is 1:(2-4).

In some embodiments, the uniform mixing is performed by adopting a microfluidic device, with a flow rate controlled to be ≥ 12 mL/min.

In a tenth aspect, the present application further relates to use of the nucleic acid molecule according to the first aspect, or the composition according to the second aspect, or the antigen peptide according to the third aspect in the preparation of a vaccine.

In some embodiments, the vaccine includes a combination vaccine and a multivalent vaccine.

In an eleventh aspect, the present application further designs a combined vaccine comprising a first vaccine and a second vaccine for sequential use, wherein the first vaccine is selected from the nucleic acid molecule according to the first aspect, the composition according to the second aspect, the antigen peptide according to the third aspect, and the vaccine according to the fourth aspect.

In some embodiments, in the combined vaccine, the second vaccine is selected from: an attenuated or inactivated vaccine, an adenovirus vaccine, an mRNA vaccine, a DNA vaccine, and a recombinant protein vaccine.

In some embodiments, the second vaccine is selected from: Zostavax, Shingrix, and NBP608.

In some embodiments, the mRNAs of the first vaccine and the second vaccine are selected from the group consisting of nucleotide sequences set forth in any two of SEQ ID NOs. 1-7.

In some embodiments, the vaccine described in the present application is suitable for sequential vaccination with one or more vaccines selected from the following, and the vaccine may be a vaccine based on any technical route, including but not limited to attenuated or inactivated vaccines, adenovirus vaccines, mRNA vaccines, DNA vaccines, recombinant protein vaccines, and the like.

In some embodiments, for a relatively complete vaccination, the number of vaccinations of the one or more vaccines required to complete the immunization may be 1, 2, 3, or 4, and an interval of 0 days, 7 days, 21 days, 28 days, 35 days, 2 months, 3 months, 4 months, 5 months, or 6 months may be selected for each vaccination.

In a twelfth aspect, the present application further relates to a method for inducing an antigen-specific immune response in a subject, comprising administering to the subject the VZV vaccine according to the fourth aspect in an amount effective to generate an antigen-specific immune response.

In some embodiments, the antigen-specific immune response includes a T cell response.

In some embodiments, the antigen-specific immune response includes a B cell response.

In some embodiments, the subject is about 5 years old or under 5 years old; the subject is between about 1 year old and about 5 years old; the subject is between about 6 months old and about 1 year old; the subject is about 6 months old or under 6 months old; or the subject is about 12 months old or under 12 months old.

In some embodiments, the subject is an elderly subject about 60 years old, about 70 years old, or older (e.g., about 60, 65, 70, 75, 80, 85, or 90 years old).

In some embodiments, the subject is a young adult aged between about 20 years and about 50 years (e.g., about 20, 25, 30, 35, 40, 45, or 50 years).

In some embodiments, the subject is a full-term infant or a premature infant.

In some embodiments, the subject is a pregnant woman.

In some embodiments, the subject is exposed to VZV, infected with VZV, or at risk of being infected with VZV.

In some embodiments, the subject is immunocompromised (has a compromised immune system, e.g., has an immune disorder or an autoimmune disorder).

### Beneficial Effects of Present Disclosure:

In the present disclosure, an mRNA sequence is designed and optimized based on an antigen peptide derived from VZV glycoprotein E. Compared with the original coding sequence of the VZV gE protein, the GC content of the optimized sequence is improved. The optimized sequence is cloned into a vector pVAX.1, and an mRNA is synthesized *in vitro* and transfected into COS7 cells. The relative expression level of the antigen peptide is significantly increased relative to that of VZV-WT; for example, the protein expression level of the mRNA encoding VZV-trunc (SEQ ID NO. 3) is 6.5 times higher than that of VZV-WT mRNA (i.e., the original VZV gE protein mRNA). Mice immunized with the VZV mRNA vaccine of the present application are capable of producing high titers of binding antibodies, and the results are higher than those of Shingrix from GSK. Mice immunized with the VZV mRNA vaccine of the present application are capable of producing high titers of specific protective antibodies, and the results are higher than those of Shingrix. Mice immunized with the VZV mRNA vaccine of the present application are capable of producing high titers of neutralizing antibodies, and the results are higher than those of Shingrix. Mice immunized with the VZV mRNA vaccine of the present application produce very strong specific T cell immunity *in vivo*.

### BRIEF DESCRIPTION OF THE DRAWINGS

FIG. 1 shows purity and size results of the VZV gE protein mRNA by Bioanalyzer analysis.
FIG. 2 is a graph showing the results of the LNP formula optimization experiment.
FIG. 3 shows the morphology of the complex of VZV gE protein mRNA and lipid nanoparticle (LNP) by transmission electron microscopy.
FIG. 4 is a graph showing the western blotting assay results for the expression of the VZV gE protein in host cells.
FIG. 5 is a graph showing the experimental results for the expression of the target antigen protein in cells detected by an FACS flow cytometer.
FIG. 6 is a graph showing the experimental results of the detection of binding antibodies in the serum of mice after secondary immunization with different VZV mRNA vaccines (3 µg) and different doses of the positive control vaccine Shingrix.
FIG. 7 is a graph showing the experimental results of the detection (FAMA method) of specific protective antibodies in the serum of mice after secondary immunization with different VZV mRNA vaccines (3 µg) and different doses of the positive control vaccine Shingrix.
FIG. 8 is a graph showing the experimental results of the detection of neutralizing antibodies in the serum of mice after secondary immunization with different VZV mRNA vaccines (3 µg) and the positive control vaccine Shingrix.
FIG. 9 is a graph showing the experimental results of the detection of specific T cells in mice after secondary immunization with different VZV mRNA vaccines (3 µg) and different doses of the positive control vaccine Shingrix.

### DETAILED DESCRIPTION

### Definitions:

Unless defined otherwise, all technical and scientific terms used herein have the same meaning as commonly understood by those of ordinary skill in the art. For the definitions and terminology in the art, professionals can refer specifically to Current Protocols in Molecular Biology (Ausubel). Abbreviations for amino acid residues refer to standard 3-letter and/or 1-letter codes used in the art to denote one of the 20 commonly used L-amino acids.

Although the numerical ranges and parameter approximations are shown in the broad scope of the present application, the numerical values shown in the specific examples are recorded as accurately as possible. However, any numerical values inherently contain certain errors necessarily resulting from the standard deviations found in their respective measurements. In addition, all ranges disclosed herein should be understood to encompass any and all subranges subsumed therein. For example, a recorded range of "1 to 10" should be considered to include any and all subranges between the minimum value of 1 and the maximum value of 10 (including the endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" should be understood as being incorporated in its entirety.

It should be understood by those skilled in the art that many different polynucleotides may encode the same polypeptide due to the degeneracy of the genetic code. It should also be understood that skilled persons may, using conventional techniques, make nucleotide substitutions that do not affect the polypeptide sequence encoded by the nucleic acid molecule to reflect the codon usage of any particular host organism in which the polypeptide is expressed. Thus, unless otherwise stated, "polynucleotides encoding the protein or immunogenic fragment of the present application" include all polynucleotide sequences that are degenerate to each other and encode the same amino acid sequence.

As used herein, the term "reference sequence", i.e., a standard sequence for alignment of homologous sequences, may be used to define the sequence of amino acid positions in a homologous protein or polypeptide sequence. For example, amino acids in a target sequence being "numbered according to the amino acid sequence of a reference sequence as the reference sequence" means that after the reference sequence and the target sequence have identical amino acid residues at as many positions as possible by introducing gaps, deleting amino acids, or the like into the reference sequence, the reference sequence is consecutively numbered in the sequence order from the nucleotide at position 1 at the 5' end, and the amino acid positions in the target sequence corresponding to those in the reference sequence by alignment are defined by the same numbering. In the present application, unless otherwise specified, the "reference sequence" is SEQ ID NO. 8. It should be understood that "the extracellular region comprises amino acids at positions 31-538 of the gE protein" means that the extracellular region comprises an amino acid sequence consisting of amino acids at positions 31 and 538 of the gE protein numbered according to the reference sequence and all amino acids therebetween, and the length of the sequence may be longer or shorter than that of the reference sequence from position 31 to position 538 (508 mers in total) due to addition or deletion mutations of amino acids. "Amino acids at positions 539-559 of the gE protein" should also be interpreted in the same manner. The term "signal peptide" refers to a short peptide chain that directs the localization or transfer of a newly synthesized protein. Under natural conditions, the signal peptide is usually located at the N-terminus of a protein precursor, and directs the ribosome to the endoplasmic reticulum, thereby allowing the continuously synthesized polypeptide to pass through the endoplasmic reticulum. The signal peptide usually comprises a positively charged N region, a hydrophobic h region, and a neutral, polar c region, and the c region comprises a slightly conserved enzyme cutting site, which is usually recognized and cleaved by a signal peptidase on the membrane after the signal peptide directs the protein to complete the localization. In an mRNA, the coding sequence of the signal peptide is usually located after the start codon of the protein it encodes.

In the present application, "N-terminal side" is used to describe the relative position relationship between two sequence fragments, one amino acid and one sequence fragment, or two amino acids in the same amino acid sequence. The "N-terminus" refers to the terminus of the amino acid sequence containing a free amino group. For example, "the signal peptide sequence is located on the N-terminal side of the extracellular region" means that, relative to the "extracellular region", the "signal peptide sequence" is closer to the N-terminus of the amino acid sequence in which they are both located. Similarly, "C-terminal side" is also used to describe the relative position relationship between two sequence fragments, one amino acid and one sequence fragment, or two amino acids in the same amino acid sequence. The "C-terminus" refers to the terminus of the amino acid sequence containing a free carboxyl group. A sequence or amino acid located on the N-terminal side or C-terminal side of a certain sequence or amino acid may be directly linked to the certain sequence or amino acid, or separated by one or more amino acid residues.

As used herein, "coding sequence" may be used to refer to a ribonucleotide sequence in a mature mRNA that can be translated into a protein, or may refer to a complementary sequence of a deoxyribonucleotide (DNA) sequence that serves as a template for transcription of the ribonucleotide (RNA) sequence. In addition, the "coding sequence" of the present application may further comprise a sequence encoding a functional nucleic acid or element, such as a polynucleotide sequence in a DNA that can be transcribed to form a 5' UTR, a 3' UTR, a poly(A) tail, or the like.

The term "5' cap" is located at the 5' end of an mRNA and comprises methylated guanosine. The methylated guanosine is linked to the 5' end of the mRNA via pyrophosphate, forming a 5',5'-triphosphate linkage with its adjacent nucleotides. There are typically three types of 5' cap structures (m7G5'ppp5'Np, m7G5'ppp5'NmpNp, and m7G5'ppp5'NmpNmpNp), which are referred to as type O, type I, and type II, respectively. Type O means that the ribose of the terminal nucleotide is unmethylated, type I means that the ribose of one terminal nucleotide is methylated, and type II means that the ribose of both terminal nucleotides is methylated. As used herein, "CleanCap AG" is used to refer to the m7G(5')ppp(5')(2'-OMeA)pG cap.

As used herein, the term "Poly(A) tail" or "PolyA sequence" refers to an uninterrupted or interrupted sequence of adenosine residues, which is typically located at the 3'-end of an RNA molecule. The Poly-A tail or Poly-A sequence is known to those skilled in the art and can be selected according to actual needs. In the mRNA, in the presence of a 3'-UTR, the Poly-A sequence is linked to the 3' end of the 3'-UTR. An uninterrupted poly-A tail is characterized by consecutive adenosine residues. The Poly-A tail may be of any length. In some embodiments, the Poly-A tail comprises or consists of at least 20, at least 30, at least 40, at least 80, or at least 100, and at most 500, at most 400, at most 300, at most 200, or at most 150 adenosines (A), in particular about 120 As. Typically, the vast majority of nucleotides in the Poly(A) tail are adenosines, and the vast majority refers to at least 75%, at least 80%, at least 85%, at least 90%, at least 95%, at least 96%, at least 97%, at least 98%, or at least 99% of the nucleotides, but the remaining nucleotides are allowed to be nucleotides other than A, e.g., U (uridine), G (guanosine), or C (cytidine). Unless otherwise specified, the "nucleotide" of the present application, in addition to referring to naturally occurring ribonucleotide or deoxyribonucleotide monomers, should also be understood herein to refer to related structural variants thereof, including derivatives and analogs, which are functionally equivalent with respect to the specific context in which the nucleotide is used, unless otherwise clearly indicated in the context. For example, the "nucleotide" refers to a deoxyribonucleotide or a ribonucleotide. The nucleotide may be a standard nucleotide (i.e., adenosine, guanosine, cytidine, thymidine, and uridine), a nucleotide isomer, or a nucleotide analog. The nucleotide analog refers to a nucleotide having a modified purine or pyrimidine base or a modified ribose moiety. The nucleotide analog may be a naturally occurring nucleotide (e.g., inosine, pseudouridine, etc.) or a non-naturally occurring nucleotide. Non-limiting examples of modifications on a sugar or base moiety of a nucleotide include addition (or removal) of acetyl, amino, carboxyl, carboxymethyl, hydroxyl, methyl, phosphoryl, and thiol groups, and substitution of carbon and nitrogen atoms of the base with other atoms (e.g., 7-deazapurine). The nucleotide analog further includes dideoxynucleotides, 2'-O-methyl nucleotides, locked nucleic acids (LNAs), peptide nucleic acids (PNAs), and morpholino oligonucleotides.

In the present application, the "peptide" has its broadest definition. As proteins themselves comprise polypeptides, the "peptide" encompasses the meaning of polypeptides having only primary structures, as well as proteins having secondary or tertiary structures. Thus, in the present application, for example, the term "antigen peptide" encompasses a peptide chain having only a primary structure that can elicit an immune response in the organism and a protein having a secondary or tertiary structure formed by cleaving, splicing, folding, and/or stacking the peptide chain, and may represent a fragment (e.g., a truncation) of a certain protein, or may represent an intact protein. Moreover, since the protein may comprise components such as sugars, lipids, etc., the antigen peptide of the present application may also encompass glycoproteins, lipoproteins, or fragments thereof; similarly, since the protein itself may have chemical modifications, the antigen peptide of the present application may also have modifications, such as phosphorylation, glycosylation, ubiquitination, nitrosylation, methylation, acetylation, etc. The term "VZV antigen peptide" may refer to any immunogenic peptide fragment of a VZV-derived protein, a partial or complete protein of a VZV-derived protein, a partial or complete protein of a mutant of a VZV-derived protein, and a peptide fragment and a truncation of the mutant, which can elicit an immune response in the organism against VZV, of the VZV-derived protein (e.g., gE protein). It should be understood that the "peptide", "antigen peptide", and "VZV antigen peptide" of the present application may comprise amino acids, and the amino acids further include unnatural amino acids, modified amino acids (e.g., with modified side chains and/or backbones), and amino acid analogs. To illustrate further, an amino acid is typically an organic acid comprising a substituted or unsubstituted amino group, a substituted or unsubstituted carboxyl group, and one or more side chains or groups, or analogs of any one of these groups. Exemplary side chains include, for example, sulfhydryl, seleno, sulfonyl, alkyl, aryl, acyl, keto, azido, hydroxyl, hydrazine, cyano, halogen, hydrazide, alkenyl, alkynyl, ether, borate, boronate, phospho, phosphoryl, phosphine, heterocyclyl, enone, imine, aldehyde, ester, thioacid, hydroxylamine, or any combination of these groups. Other representative amino acids include, but are not limited to, amino acids containing photosensitive cross-linking agents, metal-binding amino acids, spin-labeled amino acids, fluorescent amino acids, amino acids containing metals, amino acids containing novel functional groups, amino acids that interact covalently or non-covalently with other molecules, photocaged and/or photoisomerizable amino acids, radioactive amino acids, amino acids containing biotin or biotin analogs, glycosylated amino acids, amino acids modified by other carbohydrates, amino acids containing polyethylene glycol or polyethers, heavy atom-substituted amino acids, chemically cleavable and/or photocleavable amino acids, amino acids containing carbon-linked sugars, redox-active amino acids, amino acids containing amino thioacids, and amino acids containing one or more toxic moieties. The amino acids described in the present application include, but are not limited to: 20 natural amino acids and 2-aminoadipic acid (Aad), 3-aminoadipic acid (bAad), beta-alanine or beta-aminoproprionic acid (bAla), 2-aminobutyric acid (Abu), 4-aminobutyric acid or piperidinic acid (4Abu), 6-aminocaproic acid (Acp), 2-aminoheptanoic acid (Ahe), 2-aminoisobutyric acid (Aib), 3-aminoisobutyric acid (bAib), 2-aminopimelic acid (Apm), 2,4-diaminobutyric acid (Dbu), desmosine (Des), 2,2'-diaminopimelic acid (Dpm), 2,3-diaminoproprionic acid (Dpr), ethylglycine (EtGly), N-ethylasparagine (EtAsn), hydroxylysine (Hyl), allo-hydroxylysine (aHyl), 3-hydroxyproline (3Hyp), 4-hydroxyproline (4Hyp), isodesmosine (Ide), allo-isoleucine (aIle), N-methylglycine or sarcosine (MeGly), N-methylisoleucine (MeIle), 6-N-methyllysine (MeLys), N-methylvaline (MeVal), norvaline (Nva), norleucine (Nle), and ornithine (Orm).

As used in the present application, the terms "gE protein extracellular region", "gE protein transmembrane region", "gE protein intracellular region", and "gE protein signal peptide" refer to an extracellular region, a transmembrane region, an intracellular region, and a signal peptide of the VZV gE protein or a variant thereof that retains its immunogenicity, respectively, unless otherwise specified. It should be appreciated by those skilled in the art that, since the VZV gE protein is an envelope protein, it will enter the secretory pathway after being expressed from a nucleic acid and be localized on the cell membrane of a host cell or eventually form a viral envelope together with the cell membrane. At this point, the VZV gE protein can be divided into an extracellular region (extracellular portion), a transmembrane region (transmembrane portion), and an intracellular region (intracellular portion) according to its position relative to the cell membrane or the viral envelope, wherein the extracellular region is a hydrophilic segment (not comprising a signal peptide portion unless otherwise specified), the intracellular region is a hydrophobic segment, and the transmembrane region is located between the extracellular region and the intracellular region and is a helical segment. In addition, in some embodiments, the extracellular region comprises or is a partial segment or the full length of positions 31-538 relative to the reference sequence SEQ ID NO. 8. In some embodiments, the intracellular region is a hydrophobic segment comprising or being a partial segment or the full length of positions 560-623 relative to the reference sequence SEQ ID NO. 8. In some embodiments, the transmembrane region comprises or is a partial segment or the full length of positions 539-559 relative to the reference sequence SEQ ID NO. 8.

As used herein, it should be understood that in the present application, unless otherwise specified, the "uridine" of the present application encompasses natural uridine and derivatives thereof, including but not limited to: 5-methoxymethyl uridine, 5-methylthio uridine, 1-methoxymethyl pseudouridine, 5-methyl cytidine, 5-methoxy cytidine, 1-methyl-pseudouridine (N1-methyl-pseudo-UTP), pseudouridine, 1-ethyl-pseudouridine, and 5-methoxy-uridine. In some other embodiments, all or part of the nucleic acid of the present application can be replaced by a modified base, such as 1-methyl-pseudouridine or pseudouridine.

The term "N:P ratio" as used herein, also referred to as "N/P" or "N:P" in the present application, refers to the molar ratio of a protonatable nitrogen element of an ionizable cationic lipid to a phosphate group of an mRNA. The N:P ratio describes the ratio of the cationic charge of the amino group (N+) in the ionizable cationic lipid to the anionic charge of the phosphate group (PO4-) in the nucleic acid backbone, and is the basis for the complexation of the ionizable cationic lipid and the nucleic acid through electrostatic interaction. The N:P ratio is a key formula factor of the LNP, which affects the physicochemical properties of the LNP and *in vivo* drug release.

As used herein, the term "about" refers to the usual margin of error for the respective value readily known to those skilled in the art. Reference to "about" for a value or parameter herein includes embodiments that are directed to that value or parameter itself. As used herein, the term "about" when preceding a numerical value generally indicates a range of 10% above or below the numerical value. For example, "about 100" encompasses 90 and 110. In some embodiments, when the "about" is followed by an integer not greater than 10, the "about" encompasses decimals, and the integer may be obtained by rounding off the numerical value after the decimal point of these decimals. For example, "about 9" encompasses the endpoint values in the range of 8.5 to 9.5 and all numerical values therebetween. When the numerical value following the "about" is a ratio, for example, 3:1, it may refer to a ratio, e.g., between 2.5:1 and 3.5:1, or for example, 7:1, it may refer to a ratio, e.g., between 6.5:1 and 7.5:1. In the present application, unless otherwise specified that the value is an integer or an exact decimal value, the value includes any numerical value that can be rounded to obtain the value, as well as conventional margin of error allowable in the art for obtaining or using the value.

### Antigen

As used herein, an antigen is a protein that is capable of inducing an immune response (e.g., causing the immune system to produce an antibody against the antigen). Herein, unless otherwise stated, the use of the term "antigen" includes immunogenic proteins and immunogenic fragments (immunogenic fragments that induce or are capable of inducing an immune response against at least one varicella-zoster virus). It should be understood that the term "protein" includes peptides, and the term "antigen" includes antigen fragments. Other molecules may also be antigenic, such as bacterial polysaccharides or a combination of proteins and polysaccharide structures. Viral vaccine antigens described herein include viral proteins, viral protein fragments, and engineered and/or mutated proteins derived from the varicella-zoster virus.

In addition, the term "antigen" as used herein will be recognized and understood by those of ordinary skill in the art, and means a substance that can be recognized by the immune system, preferably the adaptive immune system, and is capable of triggering an antigen-specific immune response, for example, by forming antibodies and/or antigen-specific T cells as part of the adaptive immune response. The antigen may be or may comprise a peptide or protein, which may be presented by MHC to a T cell. Also included are fragments, variants, and derivatives derived from, e.g., peptides or proteins. A varicella-zoster fusion protein comprising at least one epitope.

As those skilled in the art will recognize and be familiar with, protein fragments, functional protein domains, and homologous proteins are also considered to be within the scope of the varicella-zoster virus antigen of interest, such as any protein fragment of the varicella-zoster virus or a mutant strain thereof, provided that the fragment has immunogenicity and confers a protective immune response against the varicella-zoster virus; in addition to the identical but truncated variant of the reference protein, in some embodiments, the antigen comprises 2, 3, 4, 5, 6, 7, 8, 9, 10, or more mutations, and the length of the antigen/antigen polypeptide can range from about 4, 6, or 8 amino acids to the full-length protein.

### Epitope

Epitope: The term "epitope" (also referred to in the art as "antigenic determinant") as used herein will be recognized and understood by those of ordinary skill in the art to mean T cell antigenic epitopes and B cell antigenic epitopes. The T cell antigenic epitope refers to an antigenic epitope recognized by a T-cell receptor (TCR). The epitope component is a polypeptide after protein degradation, mostly exists inside an antigen molecule, and can be recognized by the TCR only after being processed by antigen presenting cells (APCs) and bound to an MHC molecule to form a complex. Generally included are fragments preferably having a length of about 6 to about 20 or even more amino acids, e.g., fragments that are processed and presented by MHC class I molecules, preferably having a length of about 8 to about 10 amino acids, e.g., 8, 9, or 10 amino acids (or 11 or 12 amino acids), or fragments that are processed and presented by MHC class II molecules, preferably having a length of about 13 to about 20 or even more amino acids. These fragments are generally recognized by T cells in the form of a complex consisting of the peptide fragment and an MHC molecule, i.e., these fragments are generally not recognized in their natural form. B cell epitopes are generally fragments located on the outer surface of (natural) protein or peptide antigens, preferably having 5 to 15 amino acids, more preferably having 5 to 12 amino acids, even more preferably having 6 to 9 amino acids, which may be recognized by antibodies, i.e., in their natural form. Such epitopes of proteins or peptides may also be selected from any of the variants of such proteins or peptides referred to herein. Herein, an epitope may be a conformational or discontinuous epitope consisting of fragments of the protein or peptide as defined herein that are discontinuous in the amino acid sequence of the protein or peptide as defined herein, but are brought together in the three-dimensional structure, or may be a continuous or linear epitope consisting of a single polypeptide chain.

### Nucleic acid

The term "nucleic acid" or "nucleic acid molecule" will be recognized and understood by those of ordinary skill in the art. As used herein, the term "nucleic acid" or "nucleic acid molecule" preferably refers to a DNA (molecule) or an RNA (molecule). It is preferably used synonymously with the term polynucleotide. Preferably, the nucleic acid or nucleic acid molecule is a polymer comprising or consisting of nucleotide monomers covalently linked to each other by phosphodiester bonds in a sugar/phosphate backbone. The term "nucleic acid molecule" also includes modified nucleic acid molecules, e.g., DNA or RNA molecules with base modifications, sugar modifications, or backbone modifications as defined herein.

Specifically, the composition of the present application comprises (at least one) RNA having an open reading frame (ORF) encoding a varicella-zoster virus antigen (e.g., F protein). In some embodiments, the RNA is a messenger RNA (mRNA).

In some embodiments, the nucleic acid comprises at least one heterologous untranslated region (UTR). The term "untranslated region" or "UTR" or "UTR element" will be recognized and understood by those of ordinary skill in the art to mean a portion of a nucleic acid molecule, which is generally located at the 5' end or the 3' end of a coding sequence. The portion located at the 5' end is referred to as a 5' UTR, and the portion located at the 3' end is referred to as a 3' UTR. In general, the UTR is not translated into a protein; the UTR may be a portion of a nucleic acid, such as a DNA or an RNA. The UTR may comprise elements for controlling gene expression, which are also known as regulatory elements. Such regulatory elements may be ribosome-binding sites, miRNA-binding sites, etc.; the RNA (e.g., mRNA) may further comprise a 5' UTR, a 3' UTR, 3' poly(A), and/or a 5' cap analog.

In some embodiments, the 5' UTR is a heterologous UTR, i.e., a UTR found in nature that is associated with different ORFs; in another embodiment, the 5' UTR is a synthetic UTR; the 5' UTR is a region of an mRNA located upstream (5') of a start codon (the first codon of an mRNA transcript translated by a ribosome). The 5' UTR does not encode a protein. A natural 5' UTR is characterized by playing a role in translation initiation and has features like a Kozak sequence, the Kozak sequence having the consensus sequence CCR(A/G)CCAUGG; exemplary 5' UTRs also include 5' UTRs of *Xenopus laevis* or human α-globin or β-globin, human cytochrome b-245a polypeptide, hydroxysteroid (17b) dehydrogenase, tobacco etch virus, alpha-1-globin, and the like.

In some embodiments, the 3' UTR may be heterologous or synthetic; for example, the globin UTR includes a *Xenopus laevis* β-globin UTR and a human β-globin UTR; other 3' UTRs may also be 3' UTRs of CYBA (cytochrome b-245 alpha chain), rabbit β-globin, hepatitis B virus (HBV), and α-globin, and a VEEV (Venezuelan equine encephalitis virus) virus 3' UTR sequence. In some embodiments, rps9 (ribosomal protein S9) 3' UTR, and 3' UTRs of FIG4 (FIG4 phosphoinositide 5-phosphatase), gp130, DH143, human albumin hHBB (human hemoglobin subunit beta), and HBA1 (human hemoglobin subunit alpha 1) may also be used.

In some embodiments, the 3' poly(A) is also referred to as a poly(A) tail. The poly(A) tail is an mRNA region that is located downstream, e.g., directly downstream (i.e., 3'), of a 3' UTR and contains a plurality of consecutive adenosine monophosphates. The poly(A) tail may contain 10 to 300 adenosine monophosphates, or may contain 10, 20, 30, 40, 50, 60, 70, 80, 90, 100, 110, 120, 130, 140, 150, 160, 170, 180, 190, 210, 220, 230, 240, 250, 260, 270, 280, 290, or 300 adenosine monophosphates. In some preferred embodiments, the poly(A) tail contains 50-250 adenosine monophosphates, more preferably 50-100 adenosine monophosphates, and most preferably 100 adenosine monophosphates. In the relevant biological environment (e.g., in a cell or *in vivo*), the 3' poly(A) tail functions to protect the mRNA from enzymatic degradation, e.g., in the cytoplasm, and to facilitate transcription termination and/or export of the mRNA from the nucleus and translation.

In some embodiments, the RNA (e.g., mRNA) further comprises a 5' guanosine cap. The 5' guanosine cap is on a eukaryotic mRNA transcript. The 5' cap consists of an inverted 7-methylguanosine and is linked to the rest of the eukaryotic mRNA via a 5'-5' triphosphate bridge, i.e., the so-called cap0. The cap0 primarily serves as a quality control for correct mRNA processing and helps stabilize the eukaryotic mRNA. On the basis of cap0, the first nucleotide is subjected to 2'-OH methylation, which is known as cap1. In addition to cap0 and cap1, a further methylation modification may also be performed on the second nucleotide, which is known as cap2. In general, the 5'-cap may be synthesized by different synthetic routes for the 5'-capped mRNA based on enzymatic, chemical, or chemoenzymatic methods.

In some embodiments, in *in vitro* transcription, a cap analog is directly added to an *in vitro* transcription (IVT) system, and the 5' cap analog includes, but is not limited to: m⁷Gppp(2'OMeA)pG, m⁷GpppApA, m⁷GpppApC, m⁷GpppApG, m⁷GpppApU, m⁷GpppCpA, m⁷GpppCpC, m⁷GpppCpG, m⁷GpppCpU, m⁷GpppGpA, m⁷GpppGpC, m⁷GpppGpG, m⁷GpppGpU, m⁷GpppUpA, m⁷GpppUpC, m⁷GpppUpG, m⁷GpppUpU, m⁷Gpppm⁶ApG, m⁷G_{3'Ome}pppApA, m⁷G_{3'Ome}pppApC, m⁷G_{3'Ome}pppApU, m⁷G_{3'Ome}pppApG, m⁷G_{3'Ome}pppCpA, m⁷G_{3'Ome}pppCpC, m⁷G_{3'Ome}pppCpG, m⁷G_{3'Ome}pppCpU, m⁷G_{3'Ome}pppUpA, m⁷G_{3'Ome}pppUpC, m⁷G_{3'Ome}pppUpG, m⁷G_{3'Ome}pppUpU, m⁷G_{3'Ome}pppA_{2'Ome}pG, m⁷G_{3'Ome}pppA_{2'Ome}pC, m⁷G_{3'Ome}pppA_{2'Ome}pU, m⁷G_{3'Ome}pppA_{2'Ome}pA, m⁷G_{3'Ome}pppC_{2'Ome}pA, m⁷G_{3'Ome}pppC_{2'Ome}pU, m⁷G_{3'Ome}pppC_{2'Ome}pG, m⁷G_{3'Ome}pppC_{2'Ome}pC, m⁷G_{3'Ome}pppG_{2'Ome}pA, m⁷G_{3'Ome}pppG_{2'Ome}pU, m⁷G_{3'Ome}pppG_{2'Ome}pG, m⁷G_{3'Ome}pppG_{2'Ome}pC, m⁷G_{3'Ome}pppU_{2'Ome}pA, m⁷G_{3'Ome}pppU_{2'Ome}pU, m⁷G_{3'Ome}pppU_{2'Ome}pG, m⁷G_{3'Ome}pppU_{2'Ome}pC, and the like.

In some embodiments, the cap analog may also be of other structures, such as a tetramer, a pentamer, a hexamer, a heptamer, an octamer, a nonamer, a decamer, or the like. The specific sequence may be determined according to the condition of the template.

It should also be understood that the respiratory syncytial virus mRNA vaccine of the present application may comprise any 5' untranslated region (UTR) and/or any 3' untranslated region (UTR). Nucleic acids comprise a polymer of nucleotides (nucleotide monomers). Thus, nucleic acids are also referred to as polynucleotides. Nucleic acids may be or may include, for example, deoxyribonucleic acids (DNAs), ribonucleic acids (RNAs), threose nucleic acids (TNAs), glycol nucleic acids (GNAs), peptide nucleic acids (PNAs), locked nucleic acids (LNAs), ethylene nucleic acids (ENA), cyclohexenyl nucleic acids (CeNA), and/or chimeras, and/or combinations thereof.

Messenger RNA (mRNA) is any RNA that encodes a (at least one) protein (a naturally-occurring, non-naturally-occurring, or modified polymer of amino acids) and can be translated *in vitro*, *in vivo*, *in situ*, or *ex vivo* to produce the encoded protein. The skilled person will appreciate that, unless otherwise stated, nucleic acid sequences set forth in the present application may recite "T" in a representative DNA sequence, but where the sequence represents RNA (e.g., mRNA), the "T" would be replaced by "U". Thus, for any DNA disclosed and identified herein by a particular sequence identification number, an RNA (e.g., mRNA) sequence complementary to or having the same base sequence as the DNA is also disclosed, in which case each "T" of the nucleotide sequence represents "U".

### Open reading frame

An open reading frame (ORF) is a contiguous stretch of DNA or RNA that begins with a start codon (e.g., methionine (ATG or AUG)) and ends with a stop codon (e.g., TAA, TAG or TGA, or UAA, UAG, or UGA). Generally, an ORF encodes a protein. It should be understood that the sequences disclosed herein may further comprise additional elements, e.g., 5' and 3' UTRs, but that those elements, unlike the ORF, are not necessarily present in the RNA polynucleotide of the present application.

In some embodiments, the composition comprises an RNA (e.g., mRNA) comprising a nucleotide sequence having at least 80%, at least 85%, at least 90%, at least 95%, at least 98%, or 100% identity to the nucleotide sequence of any one of SEQ ID NOs. 1-3.

In some embodiments, the open reading frame is preferably codon-optimized at least in part. Codon optimization is based on the discovery that translation efficiency can be determined by different frequencies of the occurrence of transfer RNA (tRNA) in cells. Thus, if an increased degree of so-called "rare codons" is present in the coding regions of the nucleic acids of the present application as defined herein, the translation of the respective modified nucleic acid sequences is less efficient than when codons encoding relatively "common" tRNAs are present. Those skilled in the art can perform codon optimization on the sequence to be translated according to the characteristics of its *in vitro* expression system.

### Chemically modified or unmodified nucleotide

In some embodiments, the RNA (e.g., mRNA) is not chemically modified, but comprises standard ribonucleotides consisting of adenosine, guanosine, cytosine, and uridine. In some embodiments, the nucleotides and nucleosides disclosed in the present application comprise standard nucleoside residues, such as those present in the transcribed RNA (e.g., A, G, C, or U). In some embodiments, the nucleotides and nucleosides disclosed in the present application comprise standard deoxyribonucleosides, such as deoxyribonucleosides present in the DNA (e.g., dA, dG, dC, or dT);
In some embodiments, the composition of the present application comprises an RNA having an open reading frame encoding a respiratory syncytial virus antigen, wherein the nucleic acid comprises nucleotides and/or nucleosides known in the art that may be standard (unmodified) or modified. In some embodiments, the nucleotides and nucleosides of the present application include modified nucleotides or nucleosides. Such modified nucleotides and nucleosides may be naturally occurring modified nucleotides and nucleosides or non-naturally occurring modified nucleotides and nucleosides. Such modifications may include modifications at the sugar, backbone, or nucleobase portion of nucleotide and/or nucleoside that are well known in the art.

In some embodiments, the modified nucleic acid base in the nucleic acid (e.g., an RNA nucleic acid, e.g., an mRNA nucleic acid) includes 1-methyl-pseudouridine, 1-ethyl-pseudouridine, 5-methoxy-uridine, 5-methyl-cytidine, and/or pseudouridine.

### In vitro transcription system (IVT)

*In vitro* transcription involves using a DNA as a template to mimic the *in vivo* transcription process in an *in vitro* cell-free system containing components such as an RNA polymerase and NTPs to generate an mRNA. In general, the capped RNA synthesized in the *in vitro* transcription reaction may be used for subsequent assays such as microinjection, *in vitro* translation, and transfection. The *in vitro* transcription system typically comprises a transcription buffer, nucleotide triphosphates (NTPs), an RNase inhibitor, and a polymerase. NTPs may be self-synthesized or selected from suppliers. NTPs may be natural or unnatural NTPs. Optional polymerases include, but are not limited to, bacteriophage RNA polymerase, e.g., T7 RNA polymerase, T3 RNA polymerase, SP6 RNA polymerase, and/or polymerase mutants thereof, for example, but not limited to, polymerases capable of incorporating modified nucleic acids and/or modified nucleotides, including chemically modified nucleic acids and/or nucleotides. Some embodiments exclude the use of DNase. In some embodiments, the RNA comprises a 5' guanosine cap.

In addition to the synthesis in the *in vitro* transcription system, chemical synthesis methods can also be used, including solid-phase chemical synthesis and liquid-phase chemical synthesis. For solid-phase chemical synthesis, all or part of the nucleic acids disclosed in the present application can be prepared by using solid-phase techniques. Solid-phase chemical synthesis of nucleic acids is an automated method in which molecules are immobilized on a solid support and are synthesized step by step in a reactant solution. Solid-phase synthesis can be used for site-specific introduction of chemical modifications in nucleotide sequences. For liquid-phase chemical synthesis, the synthesis of the nucleic acids of the present application by sequential addition of monomer constructs can be performed in a liquid phase. In addition, the synthesis methods described above can also be used in combination, as each of the synthesis methods discussed above has its own advantages and limitations, and these methods can be tried in combination to overcome the limitations described above. Combinations of these methods are within the scope of the present application.

### Antigen variant

In some embodiments, the composition of the present application comprises an RNA encoding a varicella-zoster virus antigen variant (e.g., variant trimeric F protein, e.g., stable pre-fusion F protein). Antigen variants or other polypeptide variants refer to molecules whose amino acid sequences differ from the wild-type, natural, or reference sequence. Antigen/polypeptide variants may have substitutions, deletions, and/or insertions at certain positions within the amino acid sequence compared to the natural or reference sequence. Generally, the variant has at least 50% identity to the wild-type, natural, or reference sequence. In some embodiments, the variant has at least 80% or at least 90% identity to the wild-type, natural, or reference sequence.

Variant antigens/polypeptides encoded by the nucleic acids in the content of the present application may contain amino acid changes that confer any of a variety of desirable properties, e.g., enhancing their immunogenicity, enhancing their expression, and/or improving their stability or PK/PD properties. Variant antigens/polypeptides can generally be prepared using conventional mutagenesis techniques and analyzed to determine whether they possess the desired properties as appropriate. Assays to determine expression levels and immunogenicity are well known in the art, and examples of such assays are set forth in the Examples section. Similarly, the PK/PD properties of protein variants can be measured using techniques recognized in the art, e.g., by determining the expression of an antigen in a vaccinated subject over time and/or by observing the persistence of the induced immune response. The stability of a protein encoded by a variant nucleic acid may be measured by determining the thermal stability or stability upon urea denaturation, or may be measured using computational prediction. Methods for such assays and computational determination are known in the art.

The term "identity" refers to a relationship between the sequences of two or more polypeptides (e.g., antigens) or polynucleotides (nucleic acids), as determined by comparing the sequences. The identity also refers to the degree of sequence relatedness between the sequences, as determined by the number of matches between strings of two or more amino acid residues or nucleic acid residues. The identity measures the percentage of identical matches between the smaller sequences of two or more sequences, where gap alignments (if any) are addressed by a particular mathematical model or computer program (e.g., an "algorithm"). Identity of related antigens or nucleic acids can be readily calculated by known methods. "Percent (%) identity" for a polypeptide or polynucleotide sequence is defined as the percentage of residues (amino acid residues or nucleic acid residues) in a candidate amino acid or nucleotide sequence that are identical to the residues in a second sequence after aligning the sequences and introducing gaps, if necessary, to achieve the maximum percent identity. Methods and computer programs for alignment are well known in the art. It will be understood that the identity depends on the calculation of percent identity, but may vary in value due to gaps and penalties introduced in the calculation. Generally, a variant of a particular polynucleotide or polypeptide (e.g., antigen) has a sequence identity of 40%, 45%, 50%, 55%, 60%, 65%, 70%, 75%, 80%, 85%, 90%, 91%, 92%, 93%, 94%, 95%, 96%, 97%, 98%, 99%, or 100%, as determined by sequence alignment programs and parameters described herein for the particular reference polynucleotide or polypeptide and known to those skilled in the art.

### Lipid nanoparticle (LNP)

The RNA (e.g., mRNA) of the present application is formulated in a lipid nanoparticle (LNP). The lipid nanoparticle generally comprises an ionizable cationic lipid, a helper lipid, cholesterol, and a PEG lipid component, as well as a nucleic acid of interest. The lipid nanoparticle of the present application can be produced by using components, compositions, and methods generally known in the art.

### Multivalent vaccine

The composition provided herein may comprise an RNA or a plurality of RNAs encoding two or more antigens of identical or different species. In some embodiments, the composition comprises an RNA or a plurality of RNAs encoding two or more respiratory syncytial virus antigens. In some embodiments, the RNA may encode 1, 2, 3, 4, 5, 6, 7, 8, 9, 10, 11, 12, or more respiratory syncytial virus antigens.

Two or more different RNAs (e.g., mRNAs) encoding antigens may be formulated in the same lipid nanoparticle. In other embodiments, two or more different RNAs encoding antigens may be formulated in separate lipid nanoparticles (each RNA is formulated in a single lipid nanoparticle). Subsequently, the lipid nanoparticles may be combined and administered as a single vaccine composition (e.g., comprising a plurality of RNAs encoding a plurality of antigens), or may be administered separately.

### Combination vaccine

The composition provided herein may comprise an RNA or a plurality of RNAs encoding two or more antigens of identical or different viral strains. Also provided herein is a combination vaccine comprising an RNA encoding one or more varicella-zoster virus antigens and antigens of one or more different organisms. Thus, the vaccine of the present application may be a combination vaccine targeting one or more antigens of the same strain/species, or one or more antigens of different strains/species, such as other microorganisms found in geographic regions where the risk of varicella-zoster virus infection is high, or other antigens that an individual may come into contact with at the same time when being exposed to varicella-zoster virus.

### Sequential vaccination

Sequential vaccination refers to vaccination at intervals using different technical routes, including sequential primary immunization and sequential booster immunization; if an inactivated vaccine is used for the first dose, and an adenovirus vaccine or mRNA vaccine or any other vaccine of non-inactivated routes is used for the second dose, this mode of vaccination is referred to as sequential primary immunization; if two doses of inactivated vaccinations have been previously completed, booster vaccines are subsequently required, and any other vaccine of non-inactivated routes is used, this mode of vaccination is referred to as sequential booster immunization.

### Pharmaceutical formulation

Provided herein are a composition (e.g., pharmaceutical composition), a method, a kit, and a reagent for preventing or treating varicella-zoster virus infection, e.g., in humans and other mammals. The compositions provided herein may be used as a therapeutic agent or prophylactic agent. They can be used in medicaments for the prevention and/or treatment of a varicella-zoster virus infection.

The term "pharmaceutical composition" refers to a combination of an active agent with an inert or active carrier, making the composition particularly suitable for diagnostic or therapeutic use *in vivo* or *in vitro*. "Pharmaceutically acceptable carrier" does not cause undesirable physiological effects upon or after administration to a subject. The carrier in a pharmaceutical composition must be "acceptable" in the sense that it is compatible with the active ingredient and capable of stabilizing the active ingredient. One or more solubilizers may be used as pharmaceutical carriers for delivery of the active agent. Examples of pharmaceutically acceptable carriers include, but are not limited to, biocompatible carriers, adjuvants, additives, and diluents, to obtain compositions that may be used as dosage forms. Examples of other carriers include colloidal silica, magnesium stearate, cellulose, and sodium dodecyl sulfate. Other suitable pharmaceutical carriers and diluents, as well as pharmaceutical necessities for them, are described in Remington's Pharmaceutical Sciences.

Methods for preparing mRNA vaccines are known in the art. In particular, while the mRNA in the mRNA vaccine comprises a coding sequence of an antigen peptide, it further comprises a coding sequence for a plurality of necessary functional elements to express, regulate, or enhance the expression level of the HPV antigen polypeptide described above. The functional elements include, but are not limited to, a 5' cap, a 5' UTR, a 3' UTR, a Poly tail, and the like. The functional elements are known in the art, and may be selected and combined by those skilled in the art according to actual needs. Both 5' UTR and 3' UTR are generally transcribed from genomic DNA and are elements of the pre-mature mRNA (or referred to as mRNA precursor or pre-mRNA). Characteristic structural features of mature mRNA (e.g., 5'-cap and 3'-poly(A) tail) are generally added to the transcribed (pre-mature) mRNA during mRNA processing. Thus, in some embodiments, the mRNA is an mRNA precursor. In some embodiments, the mRNA is a mature mRNA.

Related sequences involved in the present application are as follows.

It should be understood that the following sequences are merely exemplary sequences of the embodiments of the present application, and are not intended to limit the solutions of the present application in any way. The nucleic acid sequence in the following sequences may represent a DNA sequence or an RNA sequence, and when it represents an RNA sequence, unless otherwise specified, "T" and "U" are used interchangeably to indicate uridine. The sequences used in the specific examples hereinafter correspond to the following sequences according to sequence numbers or names, and when they represent mRNA sequences in the specific examples, the uridines therein are all 1-methyl-pseudouridines.
SEQ ID NO. 1
   Original nucleotide sequence of VZV gE protein VZV-WT 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGGACAGTTAATAAACCTGTGGTGGGGGTATTGATGGGGTTCGGA
ATTATCACGGGAACGTTGCGTATAACGAATCCGGTCAGAGCATCCGTCTTGCGATACG
ATGATTTTCACACCGATGAAGACAAACTGGATACAAACTCCGTATATGAGCCTTACTA
CCATTCAGATCATGCGGAGTCTTCATGGGTAAATCGGGGAGAGTCTTCGCGAAAAGCG
TACGATCATAACTCACCTTATATATGGCCACGTAATGATTATGATGGATTTTTAGAGAA
CGCACACGAACACCATGGGGTGTATAATCAGGGCCGTGGTATCGATAGCGGGGAACG
GTTAATGCAACCCACACAAATGTCTGCACAGGAGGATCTTGGGGACGATACGGGCATC
CACGTTATCCCTACGTTAAACGGCGATGACAGACATAAAATTGTAAATGTGGACCAAC
GTCAATACGGTGACGTGTTTAAAGGAGATCTTAATCCAAAACCCCAAGGCCAAAGACT
CATTGAGGTGTCAGTGGAAGAAAATCACCCGTTTACTTTACGCGCACCGATTCAGCGG
ATTTATGGAGTCCGGTACACCGAGACTTGGAGCTTTTTGCCGTCATTAACCTGTACGGG
AGACGCAGCGCCCGCCATCCAGCATATATGTTTAAAACATACAACATGCTTTCAAGAC
GTGGTGGTGGATGTGGATTGCGCGGAAAATACTAAAGAGGATCAGTTGGCCGAAATCA
GTTACCGTTTTCAAGGTAAGAAGGAAGCGGACCAACCGTGGATTGTTGTAAACACGAG
CACACTGTTTGATGAACTCGAATTAGACCCCCCCGAGATTGAACCGGGTGTCTTGAAA
GTACTTCGGACAGAAAAACAATACTTGGGTGTGTACATTTGGAACATGCGCGGCTCCG
ATGGTACGTCTACCTACGCCACGTTTTTGGTCACCTGGAAAGGGGATGAAAAAACAAG
AAACCCTACGCCCGCAGTAACTCCTCAACCAAGAGGGGCTGAGTTTCATATGTGGAAT
TACCACTCGCATGTATTTTCAGTTGGTGATACGTTTAGCTTGGCAATGCATCTTCAGTAT
AAGATACATGAAGCGCCATTTGATTTGCTGTTAGAGTGGTTGTATGTCCCCATCGATCC
TACATGTCAACCAATGCGGTTATATTCTACGTGTTTGTATCATCCCAACGCACCCCAAT
GCCTCTCTCATATGAATTCCGGTTGTACATTTACCTCGCCACATTTAGCCCAGCGTGTTG
CAAGCACAGTGTATCAAAATTGTGAACATGCAGATAACTACACCGCATATTGTCTGGG
 
AATATCTCATATGGAGCCTAGCTTTGGTCTAATCTTACACGACGGGGGCACCACGTTAA
AGTTTGTAGATACACCCGAGAGTTTGTCGGGATTATACGTTTTTGTGGTGTATTTTAAC
GGGCATGTTGAAGCCGTAGCATACACTGTTGTATCCACAGTAGATCATTTTGTAAACGC
AATTGAAGAGCGTGGATTTCCGCCAACGGCCGGTCAGCCACCGGCGACTACTAAACCC
AAGGAAATTACCCCCGTAAACCCCGGAACGTCACCACTTCTACGATATGCCGCATGGA
CCGGAGGGCTTGCAGCAGTAGTACTTTTATGTCTCGTAATATTTTTAATCTGTACGGCT
AAACGAATGAGGGTTAAAGCCTATAGGGTAGACAAGTCCCCGTATAACCAAAGCATGT
ATTACGCTGGCCTTCCAGTGGACGATTTCGAGGACTCGGAATCTACGGATACGGAAGA
AGAGTTTGGTAACGCGATTGGAGGGAGTCACGGGGGTTCGAGTTACACGGTGTATATA
GATAAGACCCGGTGATAATAGGCTGGAGCCTCGGTGGCCATGCTTCTTGCCCCTTGGG
CCTCCCCCCAGCCCCTCCTCCCCTTCCTGCACCCGTACCCCCGTGGTCTTTGAATAAAGT
CTGAGTGGGCGGCAAAAAAAAAAAAAAAAAAAAAAAAAAAAAAAGCATATGACTAA
AAAAAAAAAAAAAAAAAAAAAAAAAAAAAAAAAAAAAAAAAAAAAAAAAAAAAAAAAA
AAAAAAAAAAAAAA
SEQ ID NO. 2
   VZV gE protein nucleotide sequence VZV-2 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGCACCGTGAACAAGCCCGTGGTGGGCGTGCTGATGGGCTTCGGC
ATCATCACCGGCACCCTGCGGATCACCAACCCCGTGCGGGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCTCCCGGAAGGC
CTACGACCACAACAGCCCCTACATCTGGCCCCGGAACGACTACGACGGCTTCCTGGAG
AACGCCCACGAGCACCACGGCGTGTACAACCAGGGCCGGGGCATCGACAGCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
 
GGCAGCGACGGCACCAGCACCTACGCCACCTTCCTGGTGACCTGGAAGGGCGACGAGA
AGACCCGCAACCCCACCCCCGCCGTGACCCCCCAGCCCCGGGGCGCCGAGTTCCACAT
GTGGAACTACCACAGCCACGTGTTCAGCGTGGGCGACACCTTCAGCCTGGCCATGCAC
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCAGCCCCCACCTGG
CCCAGCGGGTGGCCAGCACCGTGTACCAGAACTGCGAGCACGCCGACAACTACACCGC
CTACTGCCTGGGCATCAGCCACATGGAGCCCAGCTTCGGCCTGATCCTGCACGACGGC
GGCACCACCCTGAAGTTCGTGGACACCCCCGAGAGCCTGAGCGGCCTGTACGTGTTCG
TGGTGTACTTCAACGGCCACGTGGAGGCCGTGGCCTACACCGTGGTGAGCACCGTGGA
CCACTTCGTGAACGCCATCGAGGAGCGGGGCTTCCCCCCCACCGCCGGCCAGCCCCCC
GCCACCACCAAGCCCAAGGAGATCACCCCCGTGAACCCCGGCACCAGCCCCCTGCTGC
GGTACGCCGCCTGGACCGGCGGCCTGGCCGCCGTGGTGCTGCTGTGCCTGGTGATCTTC
CTGATCTGCACCGCCAAGCGGATGCGGGTGAAGGCCTACCGGGTGGACAAGAGCCCCT
ACAACCAGAGCATGTACTACGCCGGCCTGCCCGTGGACGACTTCGAGGACAGCGAGAG
CACCGACACCGAGGAGGAGTTCGGCAACGCCATCGGCGGCAGCCACGGCGGCAGCAG
CTACACCGTGTACATCGACAAGACCCGCTGATAATAGGCTGGAGCCTCGGTGGCCATG
CTTCTTGCCCCTTGGGCCTCCCCCCAGCCCCTCCTCCCCTTCCTGCACCCGTACCCCCGT
GGTCTTTGAATAAAGTCTGAGTGGGCGGCAAAAAAAAAAAAAAAAAAAAAAAAAAAA
AAAGCATATGACTAAAAAAAAAAAAAAAAAAAAAAAAAAAAAAAAAAAAAAAAAAAA
AAAAAAAAAAAAAAAAAAAAAAAAAAAAAA
SEQ ID NO. 3
   VZV gE protein nucleotide sequence VZV-trunc 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGCACAGTAAATAAACCAGTTGTTGGAGTGCTGATGGGGTTCGGC
ATCATCACCGGCACCCTGCGGATCACCAACCCCGTGCGGGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCAGCCGGAAGGC
CTACGACCACAACAGCCCCTACATCTGGCCCCGGAACGACTACGACGGCTTCCTGGAG
AACGCCCACGAGCACCACGGCGTGTACAACCAGGGCCGGGGCATCGACTCCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
 
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
GGCAGCGACGGCACCAGCACCTACGCCACCTTCCTGGTGACCTGGAAGGGCGACGAGA
AGACCCGGAACCCCACCCCCGCCGTGACCCCCCAGCCCCGGGGCGCCGAGTTCCACAT
GTGGAACTACCACAGCCACGTGTTCAGCGTGGGCGACACCTTCAGCCTGGCCATGCAC
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCTCCCCCCACCTGGC
CCAGCGGGTGGCCAGCACCGTGTACCAGAACTGCGAGCACGCCGACAACTACACCGCC
TACTGCCTGGGCATCAGCCACATGGAGCCCAGCTTCGGCCTGATCCTGCACGACGGCG
GCACCACCCTGAAGTTCGTGGACACCCCCGAGAGCCTGAGCGGCCTGTACGTGTTCGT
GGTGTACTTCAACGGCCACGTGGAGGCCGTGGCCTACACCGTGGTGAGCACCGTGGAC
CACTTCGTGAACGCCATCGAGGAGCGGGGCTTCCCCCCCACCGCCGGCCAGCCCCCCG
CCACCACCAAGCCCAAGGAGATCACCCCCGTGAACCCCGGCACCAGCCCCCTGCTGCG
GTACGCCGCCTGGACCGGCGGCCTGGCCGCCGTGGTGCTGCTGTGCCTGGTGATCTTCC
TGATCTGCTGATAATAGGCTGGAGCCTCGGTGGCCATGCTTCTTGCCCCTTGGGCCTCC
CCCCAGCCCCTCCTCCCCTTCCTGCACCCGTACCCCCGTGGTCTTTGAATAAAGTCTGA
GTGGGCGGCAAAAAAAAAAAAAAAAAAAAAAAAAAAAAAAGCATATGACTAAAAAA
AAAAAAAAAAAAAAAAAAAAAAAAAAAAAAAAAAAAAAAAAAAAAAAAAAAAAAAAAA
AAAAAAAAA
SEQ ID NO. 4
   VZV gE protein nucleotide sequence VZV-mut-Leu 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGAACAGTAAATAAACCAGTTGTTGGAGTGCTGATGGGCTTCGGC
ATCATCACCGGCACCCTGCGGATCACCAACCCCGTGCGCGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCAGCCGGAAGGC
CTACGACCACAACAGCCCCTACATCTGGCCCCGGAACGACTACGACGGCTTCCTGGAG
 
AACGCCCACGAGCACCACGGCGTGTACAACCAGGGCCGGGGCATCGACAGCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
GGCAGCGACGGCACCAGCACCTACGCCACCTTCCTGGTGACCTGGAAGGGCGACGAGA
AGACCCGGAACCCCACCCCCGCCGTGACCCCCCAGCCCCGGGGCGCCGAGTTCCACAT
GTGGAACTACCACAGCCACGTGTTCAGCGTGGGCGACACCTTCAGCCTGGCCATGCAC
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCAGCCCCCACCTGG
CCCAGCGGGTGGCCAGCACCGTGTACCAGAACTGCGAGCACGCCGACAACTACACCGC
CTACTGCCTGGGCATCAGCCACATGGAGCCCAGCTTCGGCCTGATCCTGCACGACGGC
GGCACCACCCTGAAGTTCGTGGACACCCCCGAGAGCCTGAGCGGCCTGTACGTGTTCG
TGGTGTACTTCAACGGCCACGTGGAGGCCGTGGCCTACACCGTGGTGAGCACCGTGGA
CCACTTCGTGAACGCCATCGAGGAGCGGGGCTTCCCCCCCACCGCCGGCCAGCCCCCC
GCCACCACCAAGCCCAAGGAGATCACCCCCGTGAACCCCGGCACCAGCCCCCTGCTGC
GGTACGCCGCCTGGACCGGCGGCCTGGCCGCCGTGGTGCTGCTGTGCCTGGTGATCTTC
CTGATCTGCACCGCCAAGCGGATGCGGGTGAAGGCCTACCGGGTGGACAAGAGCCCCT
ACAACCAGAGCATGTACTACGCCGGCCTGCCCGTGGACGACTTCGAGGACCTGGAGCT
GCTGGACCTGGAGGAGGAGTTCGGCAACGCCATCGGCGGCAGCCACGGCGGCAGCAG
CTACACCGTGTACATCGACAAGACCCGGTGATAATAGGCTGGAGCCTCGGTGGCCATG
CTTCTTGCCCCTTGGGCCTCCCCCCAGCCCCTCCTCCCCTTCCTGCACCCGTACCCCCGT
GGTCTTTGAATAAAGTCTGAGTGGGCGGCAAAAAAAAAAAAAAAAAAAAAAAAAAAAA
AAGCATATGACTAAAAAAAAAAAAAAAAAAAAAAAAAAAAAAAAAAAAAAAAAAAAA
AAAAAAAAAAAAAAAAAAAAAAAAAAAA
SEQ ID NO. 5
   VZV gE protein nucleotide sequence VZV-mut-Ile 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGCACAGTTAATAAACCAGTAGTTGGAGTGCTGATGGGCTTCGGG
ATCATCACCGGCACCCTGCGGATCACCAACCCCGTGCGGGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCAGCCGGAAGGC
CTACGACCACAACAGCCCCTACATCTGGCCCCGGAACGACTACGACGGCTTCCTGGAG
AACGCCCACGAGCACCACGGCGTGTACAACCAGGGCCGGGGCATCGACTCCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
GGCAGCGACGGCACCAGCACCTACGCCACCTTCCTGGTGACCTGGAAGGGCGACGAGA
AGACCCGGAACCCCACCCCCGCCGTGACCCCCCAGCCCCGGGGCGCCGAGTTCCACAT
GTGGAACTACCACAGCCACGTGTTCAGCGTGGGCGACACCTTCAGCCTGGCCATGCAC
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCAGCCCCCACCTGG
CCCAGCGGGTGGCCAGCACCGTGTACCAGAACTGCGAGCACGCCGACAACTACACCGC
CTACTGCCTGGGCATCAGCCACATGGAGCCCAGCTTCGGCCTGATCCTGCACGACGGC
GGCACCACCCTGAAGTTCGTGGACACCCCCGAGAGCCTGAGCGGCCTGTACGTGTTCG
TGGTGTACTTCAACGGCCACGTGGAGGCCGTGGCCTACACCGTGGTGAGCACCGTGGA
CCACTTCGTGAACGCCATCGAGGAGCGGGGCTTCCCCCCCACCGCCGGCCAGCCCCCC
GCCACCACCAAGCCCAAGGAGATCACCCCCGTGAACCCCGGCACCAGCCCCCTGCTGC
GGTACGCCGCCTGGACCGGCGGCCTGGCCGCCGTGGTGCTGCTGTGCCTGGTGATCTTC
CTGATCTGCACCGCCAAGCGGATGCGGGTGAAGGCCTACCGGGTGGACAAGAGCCCCT
ACAACCAGAGCATGTACTACGCCGGCCTGCCCGTGGACGACTTCGAGGACATCGAGAT
CATCGACATCGAGGAGGAGTTCGGCAACGCCATCGGCGGCAGCCACGGCGGCTCCAGC
 
TACACCGTGTACATCGACAAGACCCGGTGATAATAGGCTGGAGCCTCGGTGGCCATGC
TTCTTGCCCCTTGGGCCTCCCCCCAGCCCCTCCTCCCCTTCCTGCACCCGTACCCCCGTG
GTCTTTGAATAAAGTCTGAGTGGGCGGCAAAAAAAAAAAAAAAAAAAAAAAAAAAAAA
AGCATATGACTAAAAAAAAAAAAAAAAAAAAAAAAAAAAAAAAAAAAAAAAAAAAAA
AAAAAAAAAAAAAAAAAAAAAAAAAAAA
SEQ ID NO. 6
   VZV gE protein nucleotide sequence VZV-mut-H362E-Leu 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGAACAGTAAATAAACCAGTTGTTGGAGTGCTGATGGGCTTCGGC
ATCATCACCGGCACCCTGCGGATCACCAACCCCGTGCGCGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCAGCCGGAAGGC
CTACGACCACAACAGCCCCTACATCTGGCCCCGGAACGACTACGACGGCTTCCTGGAG
AACGCCCACGAGCACCACGGCGTGTACAACCAGGGCCGGGGCATCGACAGCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
GGCAGCGACGGCACCAGCACCTACGCCACCTTCCTGGTGACCTGGAAGGGCGACGAGA
AGACCCGGAACCCCACCCCCGCCGTGACCCCCCAGCCCCGGGGCGCCGAGTTCCACAT
GTGGAACTACCACAGCCACGTGTTCAGCGTGGGCGACACCTTCAGCCTGGCCATGGAG
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCAGCCCCCACCTGG
CCCAGCGGGTGGCCAGCACCGTGTACCAGAACTGCGAGCACGCCGACAACTACACCGC
CTACTGCCTGGGCATCAGCCACATGGAGCCCAGCTTCGGCCTGATCCTGCACGACGGC
GGCACCACCCTGAAGTTCGTGGACACCCCCGAGAGCCTGAGCGGCCTGTACGTGTTCG
 
TGGTGTACTTCAACGGCCACGTGGAGGCCGTGGCCTACACCGTGGTGAGCACCGTGGA
CCACTTCGTGAACGCCATCGAGGAGCGGGGCTTCCCCCCCACCGCCGGCCAGCCCCCC
GCCACCACCAAGCCCAAGGAGATCACCCCCGTGAACCCCGGCACCAGCCCCCTGCTGC
GGTACGCCGCCTGGACCGGCGGCCTGGCCGCCGTGGTGCTGCTGTGCCTGGTGATCTTC
CTGATCTGCACCGCCAAGCGGATGCGGGTGAAGGCCTACCGGGTGGACAAGAGCCCCT
ACAACCAGAGCATGTACTACGCCGGCCTGCCCGTGGACGACTTCGAGGACCTGGAGCT
GCTGGACCTGGAGGAGGAGTTCGGCAACGCCATCGGCGGCAGCCACGGCGGCAGCAG
CTACACCGTGTACATCGACAAGACCCGGTGATAATAGGCTGGAGCCTCGGTGGCCATG
CTTCTTGCCCCTTGGGCCTCCCCCCAGCCCCTCCTCCCCTTCCTGCACCCGTACCCCCGT
GGTCTTTGAATAAAGTCTGAGTGGGCGGCAAAAAAAAAAAAAAAAAAAAAAAAAAAAA
AAGCATATGACTAAAAAAAAAAAAAAAAAAAAAAAAAAAAAAAAAAAAAAAAAAAAA
AAAAAAAAAAAAAAAAAAAAAAAAAAAAAA
SEQ ID NO. 7
   VZV gE protein nucleotide sequence VZV-mut-H362E-Ile 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACCATGGGCACAGTTAATAAACCAGTAGTTGGAGTGCTGATGGGCTTCGGG
ATCATCACCGGCACCCTGCGGATCACCAACCCCGTGCGGGCCAGCGTGCTGCGGTACG
ACGACTTCCACACCGACGAGGACAAGCTGGACACCAACAGCGTGTACGAGCCCTACTA
CCACAGCGACCACGCCGAGAGCAGCTGGGTGAACCGGGGCGAGAGCAGCCGGAAGGC
CTACGACCACAACAGCCCCTACATCTGGCCCCGGAACGACTACGACGGCTTCCTGGAG
AACGCCCACGAGCACCACGGCGTGTACAACCAGGGCCGGGGCATCGACTCCGGCGAG
CGGCTGATGCAGCCCACCCAGATGAGCGCCCAGGAGGACCTGGGCGACGACACCGGC
ATCCACGTGATCCCCACCCTGAACGGCGACGACCGGCACAAGATCGTGAACGTGGACC
AGCGGCAGTACGGCGACGTGTTCAAGGGCGACCTGAACCCCAAGCCCCAGGGCCAGC
GGCTGATCGAGGTGAGCGTGGAGGAGAACCACCCCTTCACCCTGCGGGCCCCCATCCA
GCGGATCTACGGCGTGCGGTACACCGAGACCTGGAGCTTCCTGCCCAGCCTGACCTGC
ACCGGCGACGCCGCCCCCGCCATCCAGCACATCTGCCTGAAGCACACCACCTGCTTCC
AGGACGTGGTGGTGGACGTGGACTGCGCCGAGAACACCAAGGAGGACCAGCTGGCCG
AGATCAGCTACCGGTTCCAGGGCAAGAAGGAGGCCGACCAGCCCTGGATCGTGGTGA
ACACCAGCACCCTGTTCGACGAGCTGGAGCTGGACCCCCCCGAGATCGAGCCCGGCGT
GCTGAAGGTGCTGCGGACCGAGAAGCAGTACCTGGGCGTGTACATCTGGAACATGCGG
GGCAGCGACGGCACCAGCACCTACGCCACCTTCCTGGTGACCTGGAAGGGCGACGAGA
AGACCCGGAACCCCACCCCCGCCGTGACCCCCCAGCCCCGGGGCGCCGAGTTCCACAT
 
GTGGAACTACCACAGCCACGTGTTCAGCGTGGGCGACACCTTCAGCCTGGCCATGGAG
CTGCAGTACAAGATCCACGAGGCCCCCTTCGACCTGCTGCTGGAGTGGCTGTACGTGC
CCATCGACCCCACCTGCCAGCCCATGCGGCTGTACAGCACCTGCCTGTACCACCCCAAC
GCCCCCCAGTGCCTGAGCCACATGAACAGCGGCTGCACCTTCACCAGCCCCCACCTGG
CCCAGCGGGTGGCCAGCACCGTGTACCAGAACTGCGAGCACGCCGACAACTACACCGC
CTACTGCCTGGGCATCAGCCACATGGAGCCCAGCTTCGGCCTGATCCTGCACGACGGC
GGCACCACCCTGAAGTTCGTGGACACCCCCGAGAGCCTGAGCGGCCTGTACGTGTTCG
TGGTGTACTTCAACGGCCACGTGGAGGCCGTGGCCTACACCGTGGTGAGCACCGTGGA
CCACTTCGTGAACGCCATCGAGGAGCGGGGCTTCCCCCCCACCGCCGGCCAGCCCCCC
GCCACCACCAAGCCCAAGGAGATCACCCCCGTGAACCCCGGCACCAGCCCCCTGCTGC
GGTACGCCGCCTGGACCGGCGGCCTGGCCGCCGTGGTGCTGCTGTGCCTGGTGATCTTC
CTGATCTGCACCGCCAAGCGGATGCGGGTGAAGGCCTACCGGGTGGACAAGAGCCCCT
ACAACCAGAGCATGTACTACGCCGGCCTGCCCGTGGACGACTTCGAGGACATCGAGAT
CATCGACATCGAGGAGGAGTTCGGCAACGCCATCGGCGGCAGCCACGGCGGCTCCAGC
TACACCGTGTACATCGACAAGACCCGGTGATAATAGGCTGGAGCCTCGGTGGCCATGC
TTCTTGCCCCTTGGGCCTCCCCCCAGCCCCTCCTCCCCTTCCTGCACCCGTACCCCCGTG
GTCTTTGAATAAAGTCTGAGTGGGCGGCAAAAAAAAAAAAAAAAAAAAAAAAAAAAAA
AGCATATGACTAAAAAAAAAAAAAAAAAAAAAAAAAAAAAAAAAAAAAAAAAAAAAA
AAAAAAAAAAAAAAAAAAAAAAAAAA
 Note: U in the sequences described above may be completely (100%) or partially replaced by modified bases, such as 1-methyl-pseudouridine or pseudouridine.
SEQ ID NO. 8
   Original amino acid sequence of VZV gE protein antigen 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
HLQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
LAAVVLLCLVIFLICTAKRMRVKAYRVDKSPYNQSMYYAGLPVDDFEDSESTDTEEEFGN
AIGGSHGGSSYTVYIDKTR
SEQ ID NO. 9
   KOZAK sequence
   GCCACCATG
SEQ ID NO. 10
   pVAX.1+TEV 5' UTR sequence 
GGGAGACCCAAGCTGGCTAGCGTTTAAACTTAAGCTTGGTACCGAGCTCGGATCCACT
AGTCCAGTGTGGTGGAATTCGGGAAATAAGAGAGAAAAGAAGAGTAAGAAGAAATAT
AAGAGCCACC
SEQ ID NO. 11
   3' poly(A) sequence 
AAAAAAAAAAAAAAAAAAAAAAAAAAAAAAAAGCATATGACTAAAAAAAAAAAAAAAAA
AAAAAAAAAAAAAAAAAAAAAAAAAAAAAAAAAAAAAAAAAAAAAAAAAAAAAAAA
SEQ ID NO. 12
   3'-UTR hemoglobin-1 (hHBA1) sequence 
GCTGGAGCCTCGGTGGCCATGCTTCTTGCCCCTTGGGCCTCCCCCCAGCCCCTCCTCCC
CTTCCTGCACCCGTACCCCCGTGGTCTTTGAATAAAGTCTGAGTGGGCGGC
SEQ ID NO. 13
   VZV-trunc 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
HLQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
LAAVVLLCLVIFLIC
SEQ ID NO. 14
   VZV-mut-Ile 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
 
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
HLQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
LAAVVLLCLVIFLICTAKRMRVKAYRVDKSPYNQSMYYAGLPVDDFEDIEIIDIEEEFGNAI
GGSHGGSSYTVYIDKTR
SEQ ID NO. 15
   VZV-mut-Ile-H362E 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
ELQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
LAAVVLLCLVIFLICTAKRMRVKAYRVDKSPYNQSMYYAGLPVDDFEDIEIIDIEEEFGNAI
GGSHGGSSYTVYIDKTR
SEQ ID NO. 16
   VZV-mut-Leu 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
HLQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
 
LAAVVLLCLVIFLICTAKRMRVKAYRVDKSPYNQSMYYAGLPVDDFEDLELLDLEEEFGN
AIGGSHGGSSYTVYIDKTR
SEQ ID NO. 17
   VZV-mut-Leu-H362E 
MGTVNKPVVGVLMGFGIITGTLRITNPVRASVLRYDDFHTDEDKLDTNSVYEPYYHSDHA
ESSWVNRGESSRKAYDHNSPYIWPRNDYDGFLENAHEHHGVYNQGRGIDSGERLMQPTQ
MSAQEDLGDDTGIHVIPTLNGDDRHKIVNVDQRQYGDVFKGDLNPKPQGQRLIEVSVEEN
HPFTLRAPIQRIYGVRYTETWSFLPSLTCTGDAAPAIQHICLKHTTCFQDVVVDVDCAENTK
EDQLAEISYRFQGKKEADQPWIVVNTSTLFDELELDPPEIEPGVLKVLRTEKQYLGVYIWN
MRGSDGTSTYATFLVTWKGDEKTRNPTPAVTPQPRGAEFHMWNYHSHVFSVGDTFSLAM
ELQYKIHEAPFDLLLEWLYVPIDPTCQPMRLYSTCLYHPNAPQCLSHMNSGCTFTSPHLAQ
RVASTVYQNCEHADNYTAYCLGISHMEPSFGLILHDGGTTLKFVDTPESLSGLYVFVVYFN
GHVEAVAYTVVSTVDHFVNAIEERGFPPTAGQPPATTKPKEITPVNPGTSPLLRYAAWTGG
LAAVVLLCLVIFLICTAKRMRVKAYRVDKSPYNQSMYYAGLPVDDFEDLELLDLEEEFGN
AIGGSHGGSSYTVYIDKTR
SEQ ID NO. 18
   TEV 5' UTR
   GGGAAATAAGAGAGAAAAGAAGAGTAAGAAGAAATATAAGAGCCACC

Embodiments of the present application will be described in detail below with reference to examples, but those skilled in the art will appreciate that the following examples are only for illustrating the present application and should not be construed as limitations to the scope of the present application. Experimental procedures without specified conditions in the examples are conducted according to conventional conditions or conditions recommended by the manufacturer. Reagents or instruments without specified manufacturers used herein are conventional products that are commercially available.

### Example 1: Codon Optimization for Encoding VZV gE Protein or Antigenic Fragment or Immunogenic Variant Protein Antigen Thereof

According to the sequence of the natural coding region of the varicella-zoster virus gE protein, an antigenic fragment thereof, or an immunogenic variant protein antigen thereof, an mRNA sequence was designed. In addition to the coding region, the features of the mRNA sequence also included pVAX.1+TEV 5' UTR, hHBA1 3' UTR, and 100 polyAs. The VZV-2, VZV-trunc, VZV-mut-Leu, VZV-mut-Ile, VZV-mut-H362E-Leu, and VZV-mut-H362E-Ile mRNA sequences were all optimized in the coding region of the VZV gE protein. Compared to the original coding sequence of the VZV gE protein, the GC contents of the optimized sequences were all increased, but remained consistent in the UTR region. The results of the sequence design optimization scheme and the relative expression level experiment are shown in Table 1.

**Table 1. Relative expression level data of different codon-optimized mRNAs**

| mRNA | 5'UTR | ORF | 3'UTR | polyA sequence and subsequent elements | Relative expression level |
|---|---|---|---|---|---|
| VZV-2(SEQ ID NO.2) | pVAX.1+TE V 5'UTR | Nucleotide sequence of gE protein (optimization scheme 1) | hHBA1 3'UTR | 100A | 400% |
| VZV-trunc(SEQ ID NO.3) | pVAX.1+TE V 5'UTR | Nucleotide sequence of gE protein truncation (optimization scheme 2) | hHBA1 3'UTR | 100A | 654% |
| VZV-mut-Leu(SEQ ID NO.4) | pVAX.1+TE V 5'UTR | Nucleotide sequence of gE protein variant (optimization scheme 3) | hHBA1 3'UTR | 100A | 402% |
| VZV-mut-Ile(SEQ ID NO.5) | pVAX.1+TE V 5'UTR | Nucleotide sequence of gE protein variant (optimization scheme 4) | hHBA1 3'UTR | 100A | 462% |
| VZV-mut-H362E-Leu(SEQ ID NO.6) | pVAX.1+TE V 5'UTR | Nucleotide sequence of F protein variant (optimization scheme 5) | hHBA1 3'UTR | 100A | 444% |
| VZV-mut-H362E-Ile(SEQ ID NO.7) | pVAX.1+TE V 5'UTR | Nucleotide sequence of F protein variant (optimization scheme 6) | hHBA1 3'UTR | 100A | 431% |
| VZV-WT(SEQ ID No.1) | pVAX.1+TE V 5'UTR | Original nucleotide sequence of gE protein | hHBA1 3'UTR | 100A | 100% |

In this study, a plurality of optimized sequences were designed and cloned into the vector pVAX.1 (GenScript Biotech Corporation). With the linearized vector (customized by GenScript Biotech Corporation) as a template, the mRNAs were synthesized *in vitro* and transfected into COS7 cells. At set time points, the expression levels of the VZV gE proteins in cells were assayed by ELISA to evaluate the effect of codon optimization on mRNA expression level and stability. The expression level results are shown in Table 1. The results show that the mRNA expression level could be significantly improved by codon optimization. According to the experimental results described above, the relative expression level of VZV-trunc antigen peptide mRNA (SEQ ID NO. 3) was relatively high, and was used as a preferred mRNA sequence for the subsequent research and development of vaccines.

The *in vitro* transcription (IVT) procedures of the VZV gE protein antigen are as follows:
1) An IVT reaction system was prepared according to the instructions of an IVT kit (a kit from Vazyme, Catalog No. DD4201-P-01), that is, 10× Transcription Buffer, ATP, GTP, CTP, PseudoUTP (1-methyl-pseudouridine, Hongene Biotech, Catalog No. R5-064), a 5' cap analog (m7G(5')ppp(5')(2'OMeA)pG, Hongene Biotech, Catalog No. ON-134), water for injection, a plasmid template (a linearized plasmid with T7 promoter, the template being the DNA sequence corresponding to SEQ ID NO. 3), and Enzyme Mix were mixed.
2) The mixed reaction system was left to react at 37 °C for 40 min.
3) The reaction was terminated by adding DNase I at a corresponding ratio.
4) An equal volume of a LiCl solution at a certain concentration (7.5 M) was added for settling twice, and then the mixture was washed twice with 75% ethanol solution, dried in air at room temperature in a super clean bench, and dissolved in an appropriate amount of RNase-free H₂O.

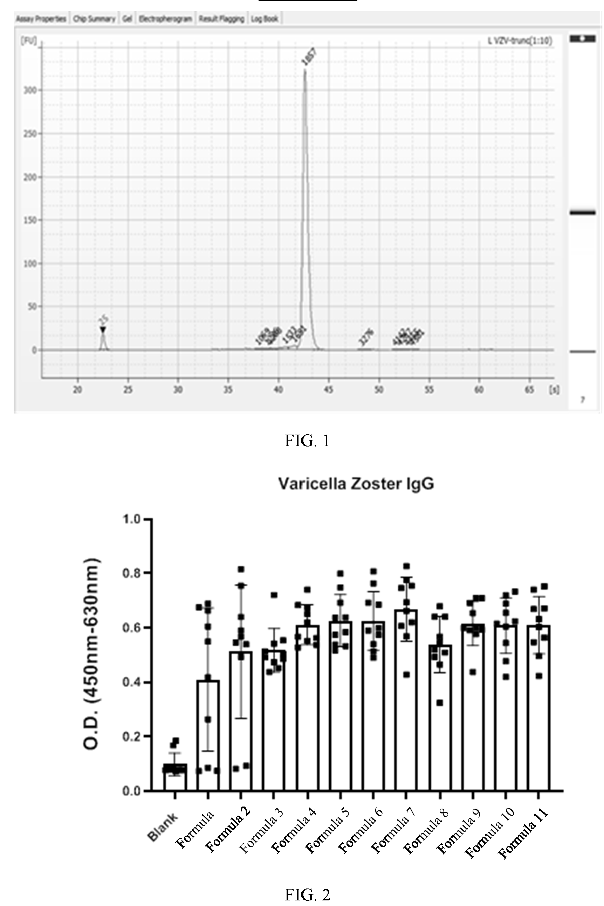

The purity of the resulting mRNA was analyzed by using Bioanalyzer. The experimental results (see FIG. 1) show that after *in vitro* synthesis of VZV-trunc antigen mRNA, a high-purity mRNA was obtained by separation and purification.

### Example 2: Formula Screening Experiment

The lipid ratio of the formulation formula was optimized through comprehensive consideration. The specific formula selections are shown in Table 2. Normal mice were immunized with a single dose of 3 µg of mRNA (encapsulated in LNPs of different formulas 1-11), and then the binding antibodies in the serum of the mice were detected on day 14. According to considerations such as binding antibody levels, formulation stability, etc., formula 9 was finally determined to be a preferred formula. FIG. 2 shows the results of LNPs encapsulating the VZV-trunc mRNA as shown in Table 1.

**Table 2. List of LNP formula components**

| No. | Lipid ratio | Treatment before administration |
|---|---|---|
| Formula 1 | HUO: 38.2%, cholesterol: 43.5%, DSPC: 15.6%, PEG-DMG: 2.6% | |
| Formula 2 | HUO: 43.8%, cholesterol: 42%, DSPC: 12.4%, PEG-DMG: 1.8% | |
| Formula 3 | HUO: 49%, cholesterol: 39%, DSPC: 8.7%, PEG-DMG: 3% | |
| Formula 4 | HUO: 49%, cholesterol: 31%, DSPC: 17%, PEG-DMG: 3% | |
| Formula 5 | HUO: 43%, cholesterol: 45%, DSPC: 8.6%, PEG-DMG: 3% | Be taken out at -20 °C, placed at room temperature, thawed, and gently shaken. |
| Formula 6 | HUO: 42.3%, cholesterol: 41%, DSPC: 14%, PEG-DMG: 2.5% | |
| Formula 7 | HUO: 40.5%, cholesterol: 50%, DSPC: 8%, PEG-DMG: 1.6% | |
| Formula 8 | HUO: 45%, cholesterol: 44%, DSPC: 9.6%, PEG-DMG: 1.4% | |
| Formula 9 | HUO: 50%, cholesterol: 38.5%, DSPC: 10%, PEG-DMG: 1.5% | |
| Formula 10 | HUO: 49%, cholesterol: 39%, DSPC: 11.5%, PEG-DMG: 1.5% | |
| Formula 11 | HUO: 49%, cholesterol: 31%, DSPC: 18.5%, PEG-DMG: 1.5% | |

| | | |
|---|---|---|
| Note: the chemical name of HUO is heptadecan-9-yl-8-((2-hydroxyethyl)(6-oxo-6-((undecyloxy)hexyl)amino)octanoate), also known as SM-102. | | |

### Example 3: Preparation and Confirmation of Antigen Peptide mRNA-LNP Formulation

The VZV gE protein or antigenic fragment or immunogenic variant protein antigen mRNA-LNP formulation was prepared according to the formula selected in Example 2 (i.e., formula 9). The specific preparation procedures are as follows:
1. Certain amounts of SM-102, DSPC, cholesterol, and DMG-PEG2000 lipid (i.e., at a molar mass ratio of 50%, 10%, 38.5%, and 1.5%, respectively) were each precisely weighed out and dissolved in an appropriate amount of absolute ethanol to prepare a lipid working solution for later use (the final concentration of the lipid working solution was 12 mg/mL).
2. A citric acid buffer (10 mM, pH 4.0) containing 130 mM sodium chloride, a Tris-NaOAc buffer (20 mM, 10.7 mM, pH 7.4), and a Tris-NaOAc buffer (20 mM, 10.7 mM, pH 7.4) containing 60% sucrose were each prepared.
3. An appropriate amount of an mRNA stock solution was diluted with the sodium chloride-citric acid buffer prepared above to adjust the final concentration of the mRNA working solution to 0.18 mg/mL.
4. The lipid working solution and the mRNA working solution were mixed in a volume ratio of 1:3 using a microfluidic instrument and a matched chip to prepare an mRNA-loaded LNP solution.
5. The Tris-NaOAc buffer was added in an amount of 9 times the volume of the prepared LNP solution for dilution, and the dilution was concentrated and purified by TFF to remove the ethanol solution from the system.
6. The mRNA content in the LNP solution was determined by the ultraviolet spectrophotometry method, and an appropriate amount of the Tris-NaOAc buffer (20 mM, 10.7 mM, pH 7.5) containing 60% sucrose was added to adjust the final concentration of the mRNA in the finished LNP solution to 100 g/mL, while the sucrose content (w/v, i.e., concentration) in the external aqueous phase system was 8.7%.

The results show that the VZV gE protein or antigenic fragment or immunogenic variant protein antigen mRNA and the lipid working solution were mixed by microfluidics to form the LNP complex with uniform particle size and morphology, mostly about 100 nm, which met the basic requirements for the next step. Illustratively, the structure of VZV-trunc mRNA-LNP is shown in FIG. 3.

### Example 4: Western Blotting Assay for Expression of VZV gE Protein Antigen Peptide in Host Cells

The mRNA expressing the VZV gE protein antigen was transfected into COS-7 cells (note: African green monkey kidney fibroblasts, purchased from Nanjing Cobioer Biosciences Co., Ltd.). 24 h after transfection, the cells were collected and subjected to the western blotting assay. Cells not transfected with the VZV gE antigen mRNA were used as a negative control (Blank), and GAPDH was used as an internal reference. FIG. 4 shows a representative result, indicating the protein expression level of VZV-trunc. It can be seen that the mRNA encoding the VZV gE antigen peptide obtained in the present application was capable of effectively and stably expressing the target antigen in COS-7 cells in large amounts.

The specific procedures of the western blotting are as follows:
1. The transfected cells were taken out, and the supernatant was discarded; the cells were washed once with PBS; 100 µL of 1× SDS-PAGE protein loading buffer was added to each well; the cells were lysed by shaking for 3 min; and the samples were collected for later use.
2. The collected samples were heated at 95 °C for 7 min, loaded, and subjected to electrophoresis at a voltage of 80 V, and the loading amount of each sample was 10 µL.
3. After the electrophoresis was completed, the methanol-activated PVDF membrane was assembled into a membrane transfer device according to the "sandwich" method, and then the membrane was transferred at a voltage of 100 V for 1.5 h in an ice bath.
4. The PVDF membrane was blocked with a PBST buffer containing 5% skim milk powder at room temperature for 1 h and washed three times with PBST, and then the PVDF membrane was incubated with VZV gE antibody at 4 °C overnight.
5. The next day, the PVDF membrane washed three times with PBST was incubated with the GAPDH antibody (mouse monoclonal antibody) at room temperature for 1 h.
6. After the unbound secondary antibody was washed away with PBST, an ECL developing solution was added, and chemiluminescence images were acquired using Proteinsimple/FluorChem E.

### Example 5: Detection of Expression of Antigen Protein in Cells by Flow Cytometry

After the mRNA expressing the VZV gE antigen peptide was transfected into COS-7 cells, the expression of the target antigen protein in the cells was detected using a flow cytometer. Cells not transfected with the VZV gE antigen peptide mRNA were used as a negative control (Blank). The specific method is described as follows:
1. To detect the expression of the VZV gE antigen peptide mRNA in cells, COS-7 cells cultured for 24 h or more were digested and transferred to a 6-well plate, with the cell density controlled to be 300,000 cells/well.
2. After the six-well plate was incubated at 37 °C for 24 h, the cell state was observed using a microscope. When the cell confluence reached 80% or more, mRNA transfection could be performed.
3. The corresponding mRNA was transfected into COS-7 cells using a Lipofectamine 2000 kit (4 µg of mRNA was transfected into each well), and the specific procedures were referred to the kit instructions. The cells were further cultured at 37 °C for 24 h.
4. After 24 h of continuous mRNA expression, the expression level was close to the peak value; at this point, the cell supernatant was removed; and the cells were washed once with PBS, digested with 0.05% trypsin for 1 min, neutralized with a complete medium, and collected. The collected cells were centrifuged at 350 g for 5 min, and the supernatant was discarded. Then the cells were resuspended in 2 mL of PBS, collected, and centrifuged at 350 g for 5 min, and the supernatant was discarded. Finally, the cells were resuspended in 100 µL of PBS, with the cell number controlled to be 200,000 to 1,000,000.
5. The collected cells were fixed with 150 µL of Cyto-Fast^{™} Fix/Perm Buffer and incubated on ice for 20 min.
6. 1 mL of 1× Cyto-Fast^{™} Perm Wash solution prepared in advance was added to the fixed cells, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
7. The primary antibody was diluted to 1:100 with 100 µL of 1× Cyto-Fast^{™} Perm Wash solution; the dilution was added to the cells; and the cells were well mixed by shaking and incubated on ice for 30 min.
8. 1 mL of 1× Cyto-Fast^{™} Perm Wash solution was added to the cells incubated with the primary antibody, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
9. The secondary antibody was diluted to 1:1,000 with 250 µL of 1× Cyto-Fast^{™} Perm Wash solution; the dilution was added to the cells; and the cells were well mixed by shaking and incubated on ice for 30 min.
10. 1 mL of 1× Cyto-Fast^{™} Perm Wash solution was added to the cells incubated with the secondary antibody, and the mixture was centrifuged at 350 g for 5 min. The supernatant was discarded, and the cells were then resuspended in 1 mL of 1× Cyto-Fast^{™} Perm Wash solution, rinsed once again, and centrifuged to discard the supernatant.
11. A 4% paraformaldehyde fixative solution was diluted to a 1% paraformaldehyde fixative solution with PBS. Finally, the cells were resuspended in 200 µL of the 1% paraformaldehyde fixative solution, and the expression of the VZV gE protein in the cells was detected on a flow cytometer.
12. Flow cytometry: Cells not transfected with the mRNA were set as a negative control group; the samples were sequentially assayed; and FITC fluorescence intensity signals were read using the histogram. 10,000 signals were read for each sample.

The experimental results show that the mRNA encoding the VZV gE antigen obtained in the present application was capable of effectively and stably expressing the target protein in COS-7 cells. For example, FIG. 5 shows the protein expression level of the antigen peptide of VZV-trunc mRNA.

### Example 6: Binding Antibodies and Assay in Serum of Mice

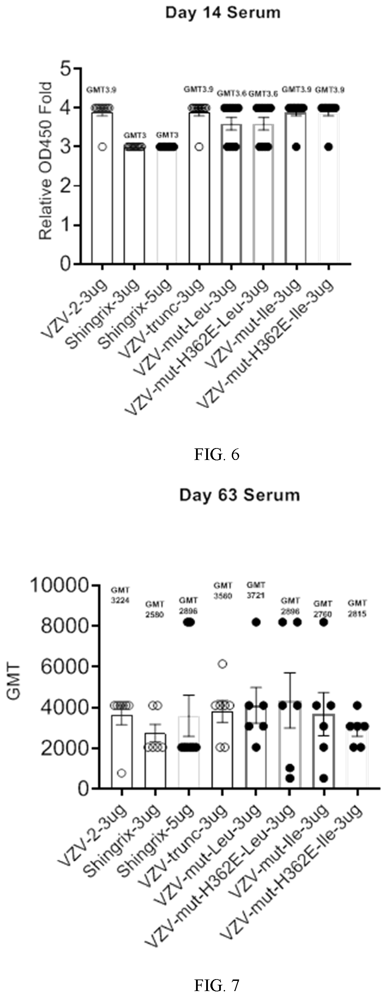

BALB/c mice aged 6-8 weeks were randomly divided into groups of 10 (half male and half female) and immunized by intramuscular injection of a vehicle, the test sample VZV mRNA-LNP formulations (3 µg/mouse), and the marketed vaccine Shingrix (3 µg/mouse or 5 µg/mouse) on day 0 and day 28. Serum samples were collected on days 14 and 35 after immunization, and the binding antibody titer in the serum on day 14 was assayed. The results in FIG. 6 show that mice immunized with the VZV mRNA vaccines of the present application were capable of producing high titers of binding antibodies, and the geometric mean titers (GMTs) of binding antibodies in the serum of mice immunized with the VZV mRNA vaccines (VZV-2, VZV-trunc, VZV-mut-Leu, VZV-mut-H362E-Leu, VZV-mut-Ile, and VZV-mut-H362E-Ile) were all higher than that of the marketed vaccine Shingrix. Among them, as compared to VZV-mut-Leu and VZV-mut-H362E-Leu, VZV-2, VZV-trunc, VZV-mut-Ile, and VZV-mut-H362E-Ile had significantly higher binding antibody GMTs in the serum, had smaller Bar values, and exhibited smaller differences in different individuals and more stable immune effects.

The method for assaying the binding antibodies in the serum of mice is shown below:
A high-binding 96-well plate coated with the VZV gE protein was prepared one day in advance. The next day, serum samples diluted to different gradients were added to the coated 96-well plate and co-incubated for 2 h, and then the binding antibodies in the serum were assayed by the universal ELISA method.

### Example 7: Specific Protective Antibody Assay in Serum of Mice (FAMA Method)

BALB/c mice aged 6-8 weeks were randomly divided into groups of 10 (half male and half female) and immunized by intramuscular injection of a vehicle, the test sample VZV gE mRNA formulations (3 µg/mouse), and the marketed vaccine Shingrix (3 µg/mouse or 5 µg/mouse) on day 0 and day 28. Serum samples were collected on days 14, 35, and 63 after immunization, and the specific protective antibody titer in the serum on day 63 was assayed. The results in FIG. 7 show that mice immunized with the VZV vaccines of the present application were capable of producing high titers of specific protective antibodies, and the geometric mean titers (GMTs) of specific protective antibodies in the serum of mice immunized with the VZV mRNA vaccines (VZV-2, VZV-trunc, VZV-mut-Leu, VZV-mut-H362E-Leu, VZV-mut-Ile, and VZV-mut-H362E-Ile) were all higher than that of the marketed vaccine Shingrix at the same dose (3 µg/mouse). Moreover, VZV-trunc and VZV-mut-Leu had significantly higher specific protective antibody GMTs than those of VZV-2, VZV-mut-H362E-Leu, VZV-mut-Ile, and VZV-mut-H362E-Ile. In addition, the Bar value of the VZV-trunc group was also smaller, indicating that it exhibits smaller differences in different individuals and a more stable immune effect.

The method for assaying the specific protective antibodies in the serum of mice is shown below:
According to the FAMA method, varicella-zoster virus (VZV)-infected cells were used as antigens to prepare immobilized antigen slides, and fluorescein isothiocyanate (FITC)-labeled goat anti-mouse IgG (H + L) was used as a secondary antibody to assay anti-VZV specific IgG antibodies in the serum of mice. The formed antigen-antibody complexes were mainly located on the surface of infected cells, and unique membranous ring-shaped fluorescence was observed under a fluorescence microscope. Those with a yellow-green fluorescent ring on the surface of infected cells were determined as positive, and those without a complete fluorescent ring or without a fluorescent ring were determined as negative. The samples were serially diluted, and the maximum dilution factor at which cells in the test group exhibited a fluorescent ring was recorded. The FAMA antibody titer was the maximum dilution factor at which the test sample exhibited a fluorescent ring.

### Example 8: Assay on Neutralizing Antibodies Against Virus in Serum of Mice

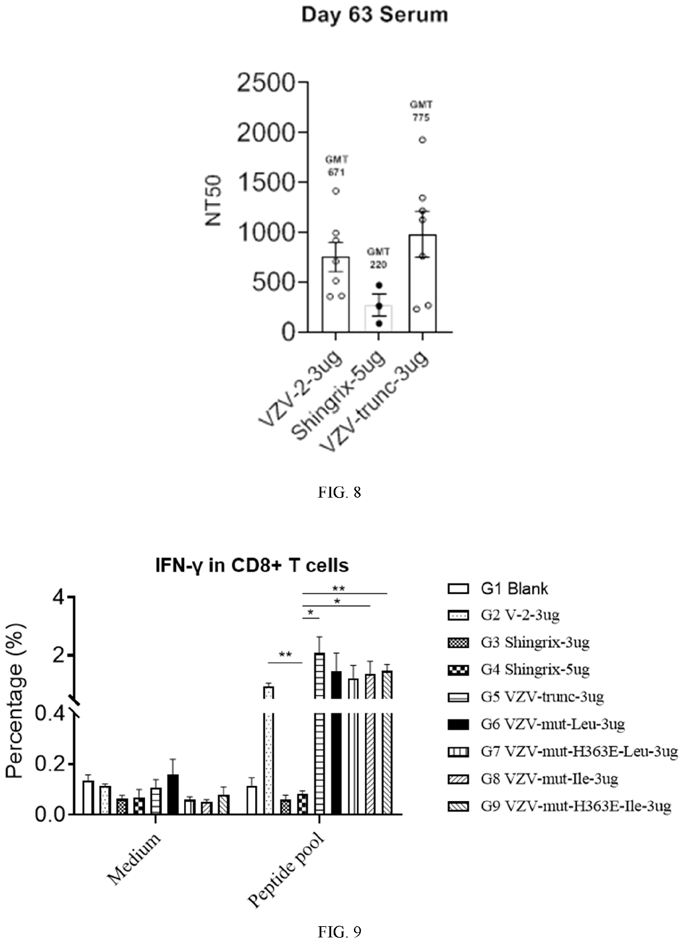

BALB/c mice aged 6-8 weeks were randomly divided into groups of 10 (half male and half female) and immunized by intramuscular injection of a vehicle, the test sample VZV mRNA-LNP formulations (3 µg/mouse), and the marketed vaccine Shingrix (3 µg/mouse or 5 µg/mouse) on day 0 and day 28. Serum samples were collected on days 14, 35, and 63 after immunization, and the neutralizing antibody titer in the serum on day 63 was assayed. The results in FIG. 8 show that mice immunized with the VZV vaccines (VZV-trunc and VZV-2) of the present application were capable of producing high titers of neutralizing antibodies, and the geometric mean titers (GMTs) of neutralizing antibodies in the serum of mice immunized with the VZV mRNA vaccines were all higher than that of the marketed vaccine Shingrix. Moreover, the neutralizing antibody GMT of VZV-trunc was significantly higher than that of VZV-2.

The method for assaying the neutralizing antibodies in the serum of mice is shown below:
1. Cell preparation: The density of MRC-5 cells was adjusted; the cells were seeded into a 96-well plate; the plate was placed in a cell incubator (37 °C, 5% CO₂) for culture overnight to ensure that the cell confluence reached about 90% the next day before starting the experiment.
2. Serum inactivation: The cells were inactivated in a water bath at 56 °C for 30 min.
3. Serial dilution: The samples were initially diluted 30-fold and subjected to 3-fold serial dilution, and a total of 8 dilutions (including the first well) were set, with 2 replicate wells.
4. Virus dilution: An appropriate amount of virus was input according to the PFU value of the virus.
5. Neutralization reaction: Diluted viruses (VR-1832^{™}, Oka strain, purchased from ATCC) were separately added to the sample wells and virus control wells, and the mixtures were sequentially diluted in a 2-fold gradient for a total of 3 dilutions in dripping wells. The mixtures were neutralized in an incubator at 37 °C with 5% CO₂ for about 1 h.
6. Virus adsorption: The viruses described above, the neutralization products in the serum, the positive viruses, and the viruses in the dripping wells were added to the cells prepared in advance at 50 µL/well, and 2 replicate wells were set. The medium was changed after the cells were cultured in an incubator at 37 °C with 5% CO₂ for about 2 h, and 100 µL of the medium was added to each well for further culture for about 48 h.
7. Plate assay: The supernatant was discarded, the cells were fixed, a fluorescently labeled assay antibody was added, and the plate was read using a CTL instrument.

### Example 9: Detection of Specific T Cell Response in Mice

Female BALB/c mice aged 6-8 weeks were randomly divided into groups of 3 and immunized by intramuscular injection of a vehicle, the test sample VZV mRNA-LNP formulations (3 µg/mouse), and the marketed vaccine Shingrix (3 µg/mouse or 5 µg/mouse) on day 0 and day 28. Spleen samples were collected on day 42 after immunization to detect the specific T cell response in mice. The results in FIG. 9 show that mice immunized with the VZV vaccines of the present application produced very strong specific T cell immunity. The proportions of specific T cells in mice in the VZV mRNA vaccine (VZV-2, VZV-trunc, VZV-mut-Leu, VZV-mut-H362E-Leu, VZV-mut-Ile, and VZV-mut-H362E-Ile) groups were significantly higher than that in the Shingrix groups at the same dose (3 µg) or even a higher dose (5 µg) (P < 0.05). Among them, the proportions of specific T cells in the VZV-trunc, VZV-mut-Leu, VZV-mut-H362E-Leu, VZV-mut-Ile, and VZV-mut-H362E-Ile groups were higher than that in the VZV-2 group; further, the proportion of specific T cells in the VZV-trunc group was also higher than those in the VZV-mut-Leu, VZV-mut-H362E-Leu, VZV-mut-Ile, and VZV-mut-H362E-Ile groups at the same dose.

The method for detecting the specific T cell response in mice is shown below:

### 1. Isolation of mouse splenocytes

The mouse spleen was ground on a 70 µm cell strainer. Dulbecco's phosphate-buffered saline (DPBS) containing 5% fetal bovine serum (FBS) was added to rinse the cells on the strainer. After centrifugation, a red blood cell lysis buffer was added for red blood cell lysis. After the lysis was completed, DPBS containing 5% FBS was added to stop the lysis. After centrifugation, the cells were resuspended in an RPMI-1640 medium containing 10% FBS, 1% GlutaMaxTM, and 1% penicillin-streptomycin (PS) and counted using a cell counter for subsequent ICS assay.

### 2. ICS assay (intracellular factor staining based on flow cytometry) of mouse splenocytes

The mouse splenocytes were seeded into a 96-well cell culture plate at 1,000,000 cells/well, and then stimuli (control group: medium; test group: VZV peptide library (PepMix^{™} VZV (gE), JPT, short peptide library covering the full length of the gE amino acid sequence) were added. The cells were incubated at 37 °C with 5% CO₂ for 16 h. A protein transport inhibitor was then added, and incubation continued for an additional 4 h. After the incubation was completed, the cells were stained in a 96-well V-well plate. Fixable Viability Stain 700 and a purified rat anti-mouse CD16/CD32 mouse Fc blocker were co-incubated at room temperature for 10 min, then CD3 and CD8 antibodies were added, and the cells were incubated at 4 °C for 30 min. The cells were washed twice with the Staining buffer, and fixed and permeabilized at 4 °C for 30 min. The cells were washed twice with the wash buffer, and intracellular cytokine staining was performed. Intracellular antibodies CD4, IFN-γ, TNF-α, and IL-2 were mixed, and the cells were incubated at 4 °C for 30 min. The cells were washed with the wash buffer, then resuspended, and transferred to a flow cytometry tube for analysis using a flow cytometer.

In conclusion, the mRNA vaccines obtained by the present application were capable of fully activating the immune system and inducing high-titer neutralizing antibodies and cellular immunity, and unexpected technical effects were achieved. In addition, VZV-trunc, VZV-mut-Leu, VZV-mut-H362E-Leu, VZV-mut-Ile, and VZV-mut-H362E-Ile all elicited significantly stronger immune responses than VZV-2. Compared with VZV-mut-Leu, VZV-mut-H362E-Leu, VZV-mut-Ile, and VZV-mut-H362E-Ile, VZV-trunc, which was the most prominent, exhibited less individual difference, elicited stronger cellular immunity, and always elicited a relatively strong humoral immune response.

## Claims

1. A nucleic acid molecule, comprising a coding sequence of a varicella-zoster virus (VZV) antigen peptide, wherein the antigen peptide comprises an extracellular region and a transmembrane region of VZV glycoprotein E (gE protein); the extracellular region comprises amino acids at positions 31-538 of the gE protein, and the transmembrane region comprises amino acids at positions 539-559 of the gE protein, the positions of the amino acids being numbered according to the amino acid sequence of SEQ ID NO. 8 as a reference sequence; and the antigen peptide comprises or has only, relative to SEQ ID NO. 8, one or more amino acid mutations selected from the following: S593L, S595L, T596L, H362E, T598L, S593I, S595I, T596I, T598I, and Del 560-623.

2. The nucleic acid molecule according to claim 1, wherein the antigen peptide comprises or has only, relative to SEQ ID NO. 8, one of the following sets of amino acid mutations:
1) S593L, S595L, T596L, and T598L;
2) H362E, S593L, S595L, T596L, and T598L;
3) H362E, S593I, S595I, T596I, and T598I;
4) S593I, S595I, T596I, and T598I; and
5) Del 560-623;
and wherein the positions of the amino acids are numbered according to the amino acid sequence of SEQ ID NO. 8 as a reference sequence.

3. The nucleic acid molecule according to claim 1 or 2, wherein the antigen peptide further comprises a signal peptide sequence, and the signal peptide sequence is located on the N-terminal side of the extracellular region.

4. The nucleic acid molecule according to claim 3, wherein the signal peptide is a signal peptide of a VZV gE protein; preferably, the signal peptide sequence comprises or is the amino acid sequence of positions 1-30 or 2-30 of SEQ ID NO. 8.

5. The nucleic acid molecule according to any one of claims 1-4, wherein the sequence of the antigen peptide is set forth in any one of SEQ ID NOs. 13-17, or the antigen peptide comprises an amino acid sequence having at least 80%, at least 85%, at least 90%, at least 95%, at least 98%, at least 99%, or 100% identity to the amino acid sequence set forth in any one of SEQ ID NOs. 13-17.

6. The nucleic acid molecule according to any one of claims 1-5, comprising a 3' poly(A) sequence or a tailing signal sequence.

7. The nucleic acid molecule according to claim 6, wherein the nucleotide sequence of the 3' poly(A) is set forth in SEQ ID NO. 11, or the 3' poly(A) comprises a nucleotide sequence having at least 100%, 99%, 98%, 97%, 96%, 95%, 90%, 85%, or 80% sequence identity to the nucleotide sequence set forth in SEQ ID NO: 11.

8. The nucleic acid molecule according to any one of claims 1-7, comprising a 5' UTR or a coding sequence of the 5' UTR, wherein the 5' UTR comprises or is a tobacco etch virus (TEV) 5' UTR.

9. The nucleic acid molecule according to claim 8, wherein the nucleotide sequence of the 5' UTR is set forth in SEQ ID NO. 18 or SEQ ID NO. 10, or the 5' UTR comprises a nucleotide sequence having at least 100%, 99%, 98%, 97%, 96%, 95%, 90%, 85%, or 80% sequence identity to the nucleotide sequence set forth in SEQ ID NO: 18 or SEQ ID NO. 10.

10. The nucleic acid molecule according to any one of claims 1-9, comprising a 3' UTR or a coding sequence of the 3' UTR, wherein the 3' UTR comprises or is a hemoglobin-1 (hHBA1) 3' UTR.

11. The nucleic acid molecule according to claim 10, wherein the nucleotide sequence of the hHBA1 3' UTR is set forth in SEQ ID NO. 12, or the hHBA1 3' UTR comprises a nucleotide sequence having at least 100%, 99%, 98%, 97%, 96%, 95%, 90%, 85%, or 80% sequence identity to the nucleotide sequence set forth in SEQ ID NO: 12.

12. The nucleic acid molecule according to any one of claims 1-11, wherein the nucleotide sequence of the nucleic acid molecule is set forth in any one of SEQ ID NOs. 2-7, or the nucleic acid molecule comprises a nucleotide sequence having at least 80%, at least 85%, at least 90%, at least 95%, at least 98%, at least 99%, or 100% sequence identity to the nucleotide sequence set forth in any one of SEQ ID NOs. 2-7.

13. The nucleic acid molecule according to any one of claims 1-12, wherein the nucleic acid molecule is an mRNA molecule.

14. The nucleic acid molecule according to any one of claims 1-13, wherein the mRNA further comprises a 5' guanosine cap selected from any one of the following: m7Gppp(2'OMeA)pG, m7GpppApA, m7GpppApC, m7GpppApG, m7GpppApU, m7GpppCpA, m7GpppCpC, m7GpppCpG, m7GpppCpU, m7GpppGpA, m7GpppGpC, m7GpppGpG, m7GpppGpU, m7GpppUpA, m7GpppUpC, m7GpppUpG, m7GpppUpU, m7Gpppm6ApG, m7G_{3'Ome}pppApA, m7G_{3'Ome}pppApC, m7G_{3'Ome}pppApU, m7G_{3'Ome}pppApG, m7G_{3'Ome}pppCpA, m7G_{3'Ome}pppCpC, m7G_{3'Ome}pppCpG, m7G_{3'Ome}pppCpU, m7G_{3'Ome}pppUpA, m7G_{3'Ome}pppUpC, m7G_{3'Ome}pppUpG, m7G_{3'Ome}pppUpU, m7G_{3'Ome}PppA_{2'Ome}pG, m7G_{3'Ome}pppA_{2'Ome}pC, m7G_{3'Ome}pppA_{2'Ome}pU, m7G_{3'Ome}pppA_{2'Ome}pA, m7G_{3'Ome}pppC_{2'Ome}pA, m7G_{3'Ome}pppC_{2'Ome}pU, m7G_{3'Ome}pppC_{2'Ome}pG, m7G_{3'Ome}pppC_{2'Ome}pC, m7G_{3'Ome}pppG_{2'Ome}pA, m7G_{3'Ome}pppG_{2'Ome}pU, m7G_{3'Ome}pppG_{2'Ome}pG, m7G_{3'Ome}pppG_{2'Ome}pC, m7G_{3'Ome}pppU_{2'Ome}pA, m7G_{3'Ome}pppU_{2'Ome}pU, m7G_{3'Ome}pppU_{2'Ome}pG, and m7G_{3'Ome}pppU_{2'Ome}pC, preferably m7G(5')ppp(5')(2'OMeA)pG.

15. The nucleic acid molecule according to claim 13 or 14, wherein one or more of uridines (U) in the nucleic acid molecule are base-modified uridines; preferably, the base-modified uridine is any one or more selected from the group consisting of 5-methoxymethyl uridine, 5-methylthio uridine, 1-methoxymethyl pseudouridine, 5-methyl cytidine, 5-methoxy cytidine, 1-methyl-pseudouridine (N1-methyl-pseudo-UTP), and pseudouridine; more preferably, each U in the nucleic acid molecule is 1-methyl-pseudouridine.

16. A composition, comprising a liposome and the nucleic acid molecule according to any one of claims 1-15.

17. The composition according to claim 16, wherein the liposome is a lipid nanoparticle (LNP), and the LNP consists of, by molar percentage (mol%), 20-60 mol% ionizable cationic lipid, 25-55 mol% structural lipid, 5-25 mol% helper lipid, and 0.5-15 mol% surfactant.

18. The composition according to claim 16 or 17, wherein the lipid nanoparticle consists of 50 mol% SM-102, 10 mol% DSPC, 38.5 mol% cholesterol, and 1.5 mol% DMG-PEG.

19. The composition according to any one of claims 16-18, wherein a cationic tertiary amine in the lipid nanoparticle and an anionic phosphate group from the nucleic acid molecule according to any one of claims 1-15 are in a molar ratio of about 3:1 to 7:1, e.g., 6:1.

20. The composition according to any one of claims 16-19, wherein the composition has a pH value comparable to a normal human physiological pH value, preferably about 7.3 to 7.5, e.g., 7.4.

21. The composition according to any one of claims 16-20, further comprising a Tris-NaOAc buffer and 8.7% sucrose.

22. The composition according to any one of claims 16-21, wherein the nucleic acid molecule is an mRNA having the sequence set forth in any one of SEQ ID NOs. 1-7, the lipid nanoparticle comprises 50 mol% SM-102, 10 mol% DSPC, 38.5 mol% cholesterol, and 1.5 mol% PEG2000-DMG, the cationic tertiary amine in the LNP and the anionic phosphate group from the nucleic acid molecule are in a molar ratio of 6:1, and the composition has a pH value of 7.4.

23. The antigen peptide according to any one of claims 1-15.

24. An engineered cell, comprising the nucleic acid molecule according to any one of claims 1-15 or the antigen peptide according to claim 23.

25. Use of the nucleic acid molecule according to any one of claims 1-15, the composition according to any one of claims 16-22, the antigen peptide according to claim 23, or the engineered cell according to claim 24 in the preparation of a vaccine.

26. A method for preventing a VZV infection or a symptom caused by VZV, comprising administering to a subject an effective amount of the nucleic acid molecule according to any one of claims 1-15, the composition according to any one of claims 16-22, the antigen peptide according to claim 23, or the engineered cell according to claim 24 to induce an immune response against the varicella-zoster virus in the subject.
